# EUROPÄISCHE PATENTSCHRIFT

(11) **EP 0 464 838 B1**
(45) Veröffentlichungstag und Bekanntmachung des Hinweises auf die Patenterteilung: **07.10.1998**
(21) Anmeldenummer: 91111204.3
(22) Anmeldetag: 05.07.1991
(51) Int. Cl.: C07D 239/52, C07D 239/47, C07D 239/42, C07D 251/14, C07D 239/48, C07D 239/56, C07D 409/12, C07D 491/048, C07D 249/14, C07D 213/75, A01N 47/36, C07D 295/22, C07D 333/48, C07D 335/02, C07D 277/34, C07D 279/02, C07C 311/03

(54) **Substituierte Sulfonylalkylsulfonylharnstoffe, Verfahren zu ihrer Herstellung und ihre Verwendung als Herbizide und Pflanzenwachstumsregulatoren**
Substituted sulfonylalkylsulfonylureas, process for their preparation and their use as herbicides and plant growth regulators
Sulfonylalkylsulfonylurées substituées, procédé pour leur préparation et leur utilisation comme herbicides et régulateurs de la croissance des plantes

(30) Priorität: 06.07.1990 DE 4021489
(43) Veröffentlichungstag der Anmeldung: 08.01.1992
(73) Patentinhaber: Hoechst Schering AgrEvo GmbH, 13342 Berlin (DE)
(72) Erfinder: Kehne, Heinz, Dr., W-6238 Hofheim am Taunus (DE); Willms, Lothar, Dr., W-5416 Hillscheid (DE); Bauer, Klaus, Dr., W-6450 Hanau (DE); Bieringer, Hermann, Dr., W-6239 Eppstein/Taunus (DE)

(56) Entgegenhaltungen:
- EP-A- 0 131 258
- EP-A- 0 252 640
- EP-A- 0 336 354
- EP-A- 0 347 788
- EP-A- 0 353 641
- US-A- 3 862 184
- US-A- 3 865 822
- US-A- 3 946 007
- 'BEILSTEINS HANDBUCH DER ORGANISCHEN CHEMIE, 4te Auflage, Ergänzungswerk 1, Band 1' 1928 , VERLAG VON JULIUS SPRINGER , BERLIN, DE
- 'BEILSTEINS HANDBUCH DER ORGANISCHEN CHEMIE, 4te Auflage, Ergänzungswerk 3, Band 1, Teil 3' 1959 , SPRINGER-VERLAG , BERLIN, DE

## Beschreibung

Es ist bekannt, daß substituierte Alkylsulfonylharnstoffe herbizide und pflanzenwachstumsregulierende Eigenschaften aufweisen (EP 61 661 (US-A-4,440,565), EP 71 958 (US-A-4,492,598), EP 85 236, EP 336 354 (ZA 89/2495)).

Die Wirkungen dieser Verbindungen sind jedoch nicht immer befriedigend.

Es wurden nun heterocyclisch substituierte Sulfonylalkylsulfonylharnstoffe gefunden, die als Herbizide und Pflanzenwachstumsregulatoren besonders geeignet sind.

Gegenstand der vorliegenden Erfindung sind daher Verbindungen der Formel (I) oder deren Salze, 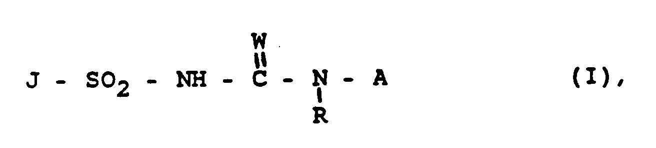 worin J einen Rest der Formel (J-1)-(J-4) 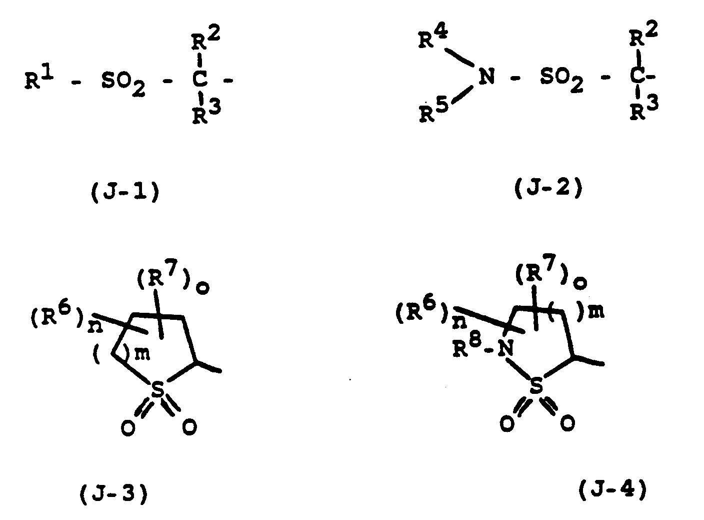 und darin
- R¹: (C₁-C₆)Alkyl, (C₂-C₆)Alkenyl, (C₂-C₆)Alkinyl, wobei die letztgenannten 3 Reste unsubstituiert oder ein- oder mehrfach durch Halogen oder ein- oder zweifach durch Reste aus der Gruppe bestehend aus (C₁-C₄)Alkoxy, (C₁-C₄)Alkylthio und (C₁-C₄)Alkoxycarbonyl substituiert sind, oder -(CH₂)ₐ-Phenyl, wobei a 0,1 oder 2 bedeutet und der Phenylrest unsubstituiert oder ein- oder mehrfach durch Reste aus der Gruppe Halogen, (C₁-C₄)Alkyl, (C₁-C₄)Alkoxy, (C₁-C₄)Alkylthio, (C₁-C₄)Alkylsulfinyl, (C₁-C₄)Alkylsulfonyl und (C₁-C₄-Alkoxy)-carbonyl und Reste analog letztgenannten 6 Resten, die im Alkylteil ein- oder mehrfach halogeniert sind, substituiert ist,
- R²,R³: unabhängig voneinander Wasserstoff, Halogen, (C₁-C₄)Alkoxy, (C₁-C₄)Alkylthio, (C₁-C₆)Alkyl, (C₂-C₆)Alkenyl, (C₂-C₆)Alkinyl, wobei die letztgenannten 5 Reste unsubstituiert oder ein- oder mehrfach durch Halogen oder ein- oder zweifach durch Reste aus der Gruppe (C₁-C₄)Alkoxy und (C₁-C₄)Alkylthio substituiert sind, (C₁-C₄-Alkoxy)-carbonyl-(C₁-C₃)-alkyl, -(CH₂)ₐ-Phenyl, wobei a 0, 1 oder 2 bedeutet und der Phenylrest unsubstituiert oder durch ein oder mehrere Reste aus der Gruppe Halogen, (C₁-C₄)Alkyl, (C₁-C₄)Alkoxy, (C₁-C₄-Alkoxy)-carbonyl, (C₁-C₄)Alkylthio und (C₁-C₄)Alkylsulfonyl, wobei die letztgenannten 5 Reste im Alkylteil unsubstituiert oder ein- oder mehrfach durch Halogen substituiert sind, substituiert ist,
- R⁴,R⁵: unabhängig voneinander Wasserstoff, (C₁-C₈)Alkyl, (C₂-C₈)Alkenyl, (C₂-C₈)Alkinyl, wobei die 3 letztgenannten Reste unsubstituiert oder ein- oder mehrfach durch Halogen oder ein- oder zweifach durch (C₁-C₄)Alkoxy oder (C₁-C₄)-Alkylthio substituiert sind, (C₁-C₄)Alkoxycarbonyl-(C₁-C₃)alkyl, -(CH₂)ₐ-Phenyl, wobei a 0, 1 oder 2 bedeutet und der Phenylrest unsubstituiert oder ein- oder mehrfach durch Reste aus der Gruppe Halogen, (C₁-C₄)Alkyl, (C₁-C₄)Alkoxy, (C₁-C₄)Alkylthio, (C₁-C₄)Alkylsulfonyl, wobei die vorgenannten 4 Reste im Alkylteil unsubstituiert oder ein- oder mehrfach durch Halogen substituiert sind, und (C₁-C₄-Alkoxy)-carbonyl substituiert ist, (C₁-C₆)Alkoxy oder Di-(C₁-C₆)alkylamino oder R⁴ und R⁵ zusammen mit dem sie verknüpfenden N-Atom einen heterocyclischen Ring der Formel 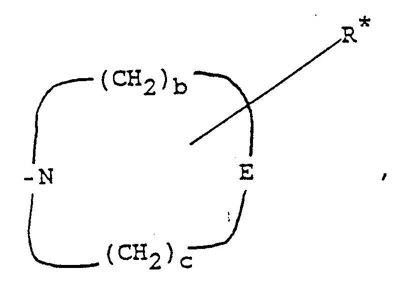 wobei b und c unabhängig voneinander 0, 1, 2 oder 3, R* Wasserstoff, (C₁-C₄)Alkyl, (C₁-C₄-Alkoxy)-carbonyl oder (C₁-C₄-Alkoxy)-methyl und E eine divalente Gruppe der Formel O, S, CH₂ oder N-(C₁-C₄)Alkyl bedeuten,
- R⁶,R⁷: unabhängig voneinander Halogen, (C₁-C₈)Alkyl, (C₂-C₈)Alkenyl, (C₂-C₈)Alkinyl oder (C₁-C₈)Alkoxy, wobei die letztgenannten 4 Reste unsubstituiert oder ein- oder mehrfach durch Halogen oder ein- oder zweifach durch (C₁-C₄)Alkoxy oder (C₁-C₄)Alkylthio substituiert sind, oder (C₁-C₄-Alkoxy)-carbonyl-(C₁-C₃)alkyl,
- R⁸: Wasserstoff, (C₁-C₈)Alkyl, (C₂-C₈)Alkenyl, (C₂-C₈)Alkinyl oder (C₁-C₈)Alkoxy, wobei die letztgenannten 4 Reste unsubstituiert oder ein- oder mehrfach durch Halogen oder ein- oder zweifach durch (C₁-C₄)Alkoxy oder (C₁-C₄)-Alkylthio substituiert sind, oder (C₁-C₄-Alkoxy)-carbonyl-(C₁-C₃)alkyl,
- R⁹: Wasserstoff, (C₁-C₈)Alkyl, (C₂-C₈)Alkenyl, (C₂-C₈)Alkinyl oder (C₁-C₈)Alkoxy, wobei die letztgenannten 4 Reste unsubstituiert oder ein- oder mehrfach durch Halogen oder ein- oder zweifach durch (C₁-C₄)Alkoxy oder (C₁-C₄)-Alkylthio substituiert sind, oder (C₁-C₄-Alkoxy)-carbonyl-(C₁-C₃)alkyl,
- R¹⁰: Wasserstoff, (C₁-C₄)Alkyl, (C₁-C₄-Alkoxy)-carbonyl oder (C₁-C₄-Alkoxy)-methyl,
- m: 1, 2 oder 3, vorzugsweise 1 oder 2,
- n,o: unabhängig voneinander 0, 1, 2 oder 3, vorzugsweise 0 oder 1,
- A: einen Rest der Formeln (A-1) bis (A-8) 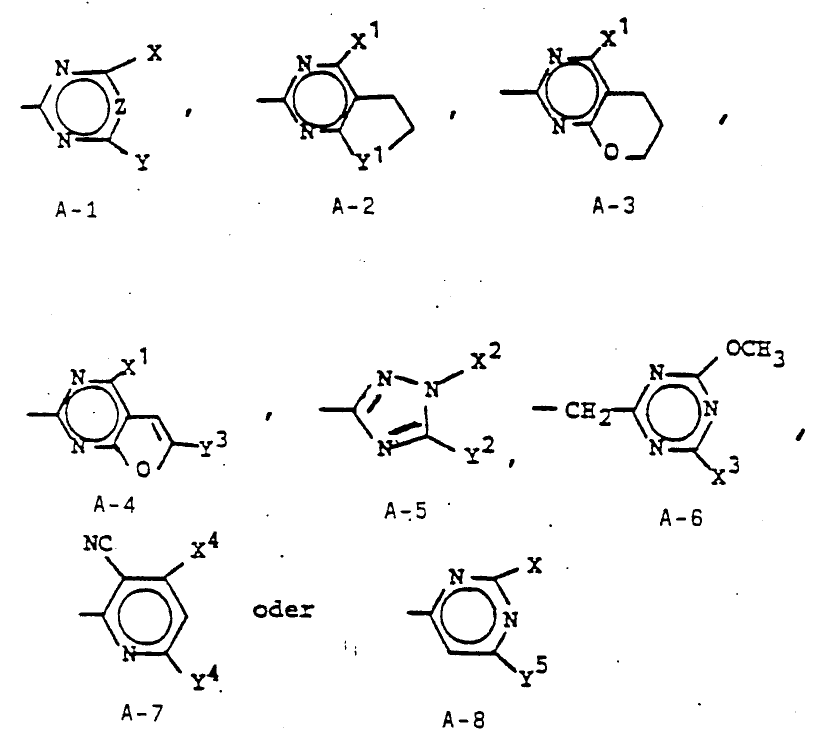
- X,Y H,: Halogen, (C₁-C₃)Alkyl, (C₁-C₃)Alkoxy oder (C₁-C₃)Alkylthio, wobei die vorgenannten alkylhaltigen Reste ein- oder mehrfach durch Halogen oder ein- oder zweifach durch (C₁-C₃)Alkoxy oder (C₁-C₃)Alkylthio substituiert sein können, ferner einen Rest der Formel NR⁹R¹⁰, (C₃-C₆)Cycloalkyl, (C₂-C₄)Alkenyl, (C₂-C₄)Alkinyl, (C₃-C₄)Alkenyloxy oder (C₃-C₄)Alkinyloxy, vorzugsweise mit der Maßgabe, daß nicht beide Reste zugleich Reste aus der Gruppe Halogen, Alkylthio, Reste der Formel NR⁹R¹⁰, Alkenyl und Alkinyl bedeuten,
- Z: CH oder N, vorzugsweise CH,
- X¹: CH₃, OCH₃, OC₂H₅ oder OCF₂H,
- Y¹: -O- oder -CH₂-,
- X²: CH₃, C₂H₅ oder CH₂CF₃,
- Y²: OCH₃, OC₂H₅, SCH₅, SC₂H₅, CH₃ oder C₂H₅,
- X³: CH₃ oder OCH₃,
- Y³: H oder CH₃,
- X⁴: CH₃, OCH₃, OC₂H₅, CH₂OCH₃ oder Cl,
- Y⁴: CH₃, OCH₃, OC₂H₅ oder Cl,
- Y⁵: CH₃, C₂H₅, OCH₃ oder Cl,
- W: O oder S und
- R: Wasserstoff oder CH₃
bedeuten.

In der Formel (I) können Alkyl-, Alkoxy-, Haloalkyl-, Alkylamino- und Alkylthioreste sowie die entsprechenden ungesättigten und/oder substituierten Reste im Alkylteil jeweils geradkettig oder verzweigt sein. Alkylreste, auch in den zusammengesetzten Bedeutungen wie Alkoxy, Haloalkyl, Alkylthio, Alkoxycarbonyl usw., bedeuten, wenn nicht näher spezifiziert, vorzugsweise Methyl, Ethyl, n- oder i-Propyl, n-, i-, t- oder 2-Butyl, Pentyl wie n- oder i-Pentyl, Hexyl wie n-, i- und 2-Hexyl; Cycloalkyl bedeutet in der Regel C₃-C₆-Cycloalkyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet Fluor, Chlor, Brom oder Jod.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metallsalze, insbesondere Alkali- oder Erdalkalisalze, oder auch Ammoniumsalze oder Salze mit organischen Aminen.

Die Sulfonylharnstoffe der Formel (I), welche im aliphatischen Rest J ein oder mehrere asymmetrische Kohlenstoffatome enthalten, können in verschiedenen enantiomeren oder diastereomeren Formen vorliegen. In der Regel werden die entsprechenden erfindungsgemäßen Verbindungen als Racemate oder als Diasteromerengemische erhalten. Falls gewünscht, können die üblichen Techniken zur Trennung dieser Gemische in die stereochemisch einheitlichen Bestandteile angewendet werden. Auch durch Verwendung von stereochemisch einheitlichen Ausgangsmaterialien ist eine Reindarstellung der genannten Verbindungen, möglich.

Die Formel (I) umfaßt daher alle obengenannten enantiomeren und diastereomeren Formen der oben definierten Verbindungen.

Bevorzugte Verbindungen der Formel (I) sind solche, bei denen R¹ = (C₁-C₄)Alkyl, R² und R³ unabhängig voneinander Wasserstoff, (C₁-C₄)Alkyl oder (C₂-C₄)Alkenyl, R⁴ und R⁵ unabhängig voneinander Wasserstoff, (C₁-C₄)Alkyl, (C₁-C₄)Alkoxy, (C₂-C₄)Alkenyl oder R⁴ und R⁵ zusammen einen heterocyclischen Rest der Formel 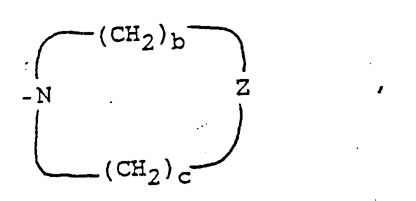 wobei b und c unabhängig voneinander 0, 1, 2 oder 3, die Summe von b+c die Zahl 3 oder 4 und Z O oder CH₂ sind, R⁶ und R⁷ unabhängig voneinander (C₁-C₃)Alkyl, R⁸ (C₁-C₄)Alkyl oder (C₂-C₄)Alkenyl, R⁹ und R¹⁰ unabhängig voneinander Wasserstoff oder (C₁-C₄)Alkyl, m 1 oder 2, n und o unabhängig voneinander 0, 1 oder 2, vorzugsweise 0 oder 1, A einen Rest der Formel (A-1), X und Y unabhängig voneinander Halogen, (C₁-C₄)Alkyl oder (C₁-C₄)Alkoxy, wobei die beiden letztgenannten Reste ein- oder mehrfach durch Halogen substituiert sein können, und W ein Sauerstoffatom bedeuten.

Bevorzugte Verbindungen der Formel (I) sind auch solche, in denen J ein Rest der genannten Formel J-2 ist. Verbindungen der Formel (I), die eine Kombination der obengenannten bevorzugten Merkmale enthalten, sind ebenfalls bevorzugt.

Weiterer Gegenstand der vorliegenden Erfindung sind Verfahren zur Herstellung von Verbindungen der obengenannten Formel (I) oder deren Salzen, dadurch gekennzeichnet, daß man
a) eine Verbindung der Formel (II),

   J - SO₂NH₂ (II)

   worin J wie in Formel (I) definiert ist,
   mit einem heterocyclischen (Thio)-Carbamat der Formel (III) umsetzt, 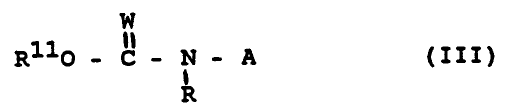 wobei R¹¹ = (C₁-C₆)Alkyl, (C₁-C₄)Halogenalkyl oder Phenyl, das unsubstituiert oder ein- oder mehrfach durch Reste aus der Gruppe Halogen, (C₁-C₄)Alkyl und NO₂ substituiert ist,
   bedeutet, und A, R und W die Bedeutungen wie bei Formel (I) haben,
b) ein Sulfonylcarbamat der Formel (IV), 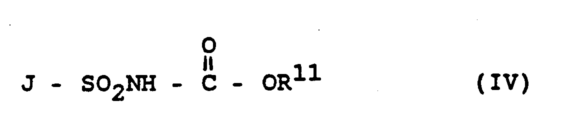 worin R¹¹ wie unter a) definiert ist,
   mit einem Aminoheterocyclus der Formel (V), 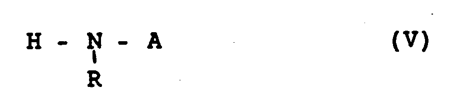 in welcher A und R wie in Formel (I) definiert sind, oder
c) ein Sulfonylisocyanat der Formel (VI),

   J - SO₂NCO (VI)

   in welcher J wie in Formel (I) definiert ist,
   mit einem Aminoheterocyclus der Formel (V) umsetzt.

Die Umsetzung der Verbindungen der Formeln (II) und (III) erfolgt in der Regel basenkatalysiert in einem inerten Lösungsmittel wie z. B. Acetonitril, Dioxan, Tetrahydrofuran oder Dichlormethan bei Temperaturen zwischen 0°C und dem Siedepunkt des Lösungsmittels. Als Base eignet sich beispielsweise 1,8-Diazabicyclo-[5.4.0.]-undec-7-en (DBU) oder - im Falle der Alkylcarbamate - Trimethylaluminium (s. EP 70 698).

Die Sulfonamide (II) sind neue Verbindungen. Sie und ihre Herstellung sind ebenfalls Gegenstand dieser Erfindung. Man erhält sie beispielsweise ausgehend von den bekannten Verbindungen des Typs J-H durch Abstraktion eines α-Protons mit einer starken Base wie z. B. Butyllithium, Umsetzung des Carbanions mit SO₂ zum Sulfinat und Aminierung, z.B. mittels Hydroxylamin-O-sulfonsäure (analog zu Synthesis 1986, 1031):

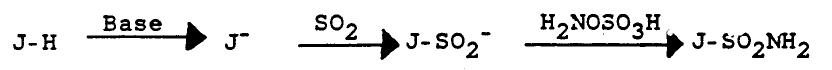

Die Carbamate der Formel (III) sind literaturbekannt oder können nach bekannten Verfahren hergestellt werden (EP 70 804 (US-A-4,443,243), RD 275 056).

Die Umsetzung der Verbindungen (IV) mit den Aminoheterocyclen (V) führt man beispielsweise in inerten, aprotischen Lösungsmitteln wie z. B. Dioxan, Acetonitril oder Tetrahydrofuran bei Temperaturen zwischen 0°C und der Siedetemperatur des Lösungsmittels durch. Die benötigten Ausgangsmaterialien (V) sind literaturbekannt oder können nach literaturbekannten Verfahren hergestellt werden. Die Sulfonylcarbamate der Formel (IV) erhält man analog EP-A-44 808 (US-A-4,419,121) oder EP-A-237 292.

Die Umsetzung der Verbindungen der Formeln (VI) und (V) erfolgt beispielsweise in inerten, aprotischen Lösungsmitteln wie z. B. Acetonitril, Dichlormethan, Toluol, Tetrahydrofuran oder Dioxan bei Temperaturen zwischen 0°C und der Siedetemperatur des Lösungsmittels.

Die Alkylsulfonyliso-(thio)-cyanate der Formel (VI) lassen sich nach im Prinzip bekannten Verfahren aus den entsprechenden Sulfonamiden der Formel (II) in einfacher Weise herstellen (vgl. z. B. EP 85 276, EP 336 354).

Die Salze der Verbindungen der Formel (I) können beispielsweise in inerten Lösungsmitteln wie z.B. Wasser, Methanol oder Aceton bei Temperaturen von 0 - 100°C hergestellt werden. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Hennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B.. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltstoffen und zur Ernteerleichterung wie z.B. durch Auslösen von Desikkation und Wuchstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) oder deren Salze enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate (SL), emulgierbare Konzentrate (EC), Emulsionen (EW), wie Öl-in-Wasser- und Wasser-in-Öl-Emulsionen, versprühbare Lösungen oder Emulsionen, Suspensionskonzentrate (SC), Dispersionen auf Öl- oder Wasserbasis, ölmischbare Lösungen (OL) Suspoemulsionen, Kapselsuspensionen (CS), Stäubemittel (DP), Beizmittel, Granulate für die Boden- bzw. Streuapplikation (FG), Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Valkenburg, "Pesticides Formulations", Marcel Dekker N.Y., 1973; K. Martens, "Spray Drying Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v. Olphen, "Introduction to Clay Colloid Chemistry"; 2nd Ed., J. Wiley & Sons, N.Y.; Marsden, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxyethylierte Alkylphenole, polyoxethylierte Fettalkohole und Fettamine, Fettalkoholpolyglykolethersulfate, Alkansulfonate oder Alkylarylsulfonate, und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salse wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Alkylpolyglycolether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophillit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Teller-, Fließbett-, Extruder- und Sprühgranulate können nach üblichen Verfahren hergestellt werden; siehe z.B. Verfahren in "Spray-Dyring Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, s. 8-57.

Für weitere Informationen zur Formulierung von Pflanzenschutzmitteln siehe z.B. G.C. Klingman, "Weed Control as a Science", John Wiley and Sons, Inc., New York, 1961, Seiten 81-96 und J.D. Freyer, s.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gewichtsprozent, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 80 Gew.-% betragen. Staubförmige Formulierungen enthalten meistens 1 bis 20 Gew.-% an Wirkstoff, versprühbare Lösungen etwa 0,2 bis 20 Gew.-%. Bei Granulaten hängt der Wirkstoffgehalt zum Teil davon ab, ob die wirksame Verbindung flüssig oder fest vorliegt. Meist liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations- oder Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen, wie Temperatur, Feuchtigkeit, der Art des verwendeten Herbizids u.a., variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Auch Mischungen oder Mischformulierungen mit anderen Wirkstoffen, wie z.B. Insektiziden, Akariziden, Herbiziden, Safenern, Düngemitteln, Wachstumsregulatoren oder Fungiziden sind gegebenenfalls möglich. Solche als Kombinationspartner geeignete Wirkstoffe sind beispielsweise aus "The Pesticide Manual" 9th Edition, British Crop Protection Council 1991 und dort zitierter Literatur bekannt.

Folgende Beispiele dienen zur Erläuterung der Erfindung:

### CHEMISCHE BEISPIELE

### Beispiel 1:

### 3-(4,6-Dimethoxypyrimidin-2-yl)-1-(1-pyrrolidinosulfonylethylsulfonyl)-harnstoff

a) 1-Pyrrolidinosulfonylethansulfonamid
   Zu 16,3 g (0,1 mol) 1-Ethylsulfonylpyrrolidin in 150 ml absolutem Tetrahydrofuran tropft man unter N₂-Atmosphäre bei -78°C 68,8 ml (0,11 mol) einer 1,6 N Lösung von n-Butyllithium in Hexan. Nach 6 Stunden bei -78°C leitet man bei derselben Temperatur 26,0 g (0,4 mol) Schwefeldioxid ein, läßt anschließend auf Raumtemperatur kommen und rührt 14 Stunden nach. Durch Eindampfen der Reaktionslösung erhält man rohes Sulfinat, das ohne Reinigung in 300 ml Wasser gelöst wird. Man gibt nacheinander 18,0 g (0,22 mol) Natriumacetat in 20 ml Wasser und 12,4 g (0,11 mol) Hydroxylamin-O-sulfonsäure in 10 ml Wasser zu und rührt 7 Stunden bei Raumtemperatur. Die Wasserphase wird mit Ethylacetat extrahiert, die organische Phase getrocknet und eingedampft. Das so erhaltene Rohprodukt nimmt man zur Reinigung in 200 ml 2 N Natronlauge auf, wäscht die Wasserphase 3 x mit je 100 ml Diethylether, säuert die Wasserphase mit Salzsäure bis pH 2 an und extrahiert mit Ethylacetat. Nach Trocknen der organischen Phase und Eindampfen hinterbleiben 12,8 g (53 % d. Th.) 1-Pyrrolidinosulfonylethansulfonamid vom Schmp. 78 - 80°C.
b) 3-(4,6-Dimethoxypyrimidin-2-yl)-1-(1-pyrrolidinosulfonylethylsulfonyl)-harnstoff
   Zu 3,6 g (0,015 mol) 1-Pyrrolidinosulfonylethansulfonamid (Beispiel 1a) und 4,0 g (0,019 mol) Phenyl-4,6-dimethoxypyrimidin-2-ylcarbamat in 40 ml Acetonitril gibt man 2,9 g (0,019 mol) 1,8-Diazabicyclo-[5.4.0.]-undec-7-en (DBU).
   Nach fünfstündigem Rühren bei Raumtemperatur versetzt man mit 250 ml Eis/Wasser und säuert mit HCl auf pH 2 an. Extraktion mit Dichlormethan, Trocknen und Eindampfen liefert rohen 3-(4,6-Dimethoxypyrimidin-2-yl)-1-(1-pyrrolidinosulfonylethylsulfonyl)-harnstoff, der durch Verreiben mit Diisopropylether gereinigt wird (Ausbeute: 4,6 g; 73 % d. Th.; Schmp. 145 - 146°C).

### Beispiel 2:

### 3-(4-Methoxy-6-methyl-1,3,5-triazin-2-yl)-1-(1-pyrrolidinosulfonylethylsulfonyl)-harnstoff

Zu 3,6 g (0,015 mol) 1-Pyrrolidinosulfonylethansulfonamid (Beispiel 1a) in 150 ml Dichlormethan tropft man bei Raumtemperatur 9 ml (0,018 mol) einer 2 M Lösung von Trimethylaluminium in Toluol. Nach Beendigung der Gasentwicklung wird 3,6 g (0,018 mol) Methyl-4-methoxy-6-methyl-1,3,5-triazin-2-ylcarbamat in 30 ml Dichlormethan zugetropft und die resultierende Lösung 24 Stunden zum Rückfluß erhitzt. Man kühlt ab und gießt in 150 ml eiskalte 1 N Salzsäure. Die organische Phase wird abgetrennt und die Wasserphase 2 x mit Dichlormethan extrahiert. Man trocknet die organische Phase und dampft ein. Nach Verreiben des Rohprodukts mit Diethylether erhält man 2,9 g (47 % d. Th.) 3-(4-Methoxy-6-methyl-1,3,5-triazin-2-yl)-1-(1-pyrrolidinosulfonylethylsulfonyl)-harnstoff vom Schmp. 136 - 137°C.

### Beispiel 3

### 3-(4-Chlor-6-methoxypyrimidin-2-yl)-1-(1-pyrrolidinosulfonylethylsulfonyl)-harnstoff

a) 3-Butyl-1-(1-pyrrolidinosulfonylethylsulfonyl)-harnstoff 3,6 g (0,015 mol) 1-Pyrrolidinosulfonylethansulfonamid (Beispiel 1a) werden in 50 ml Aceton gelöst und nach Zugabe von 2,5 g (0,018 mol) Kaliumcarbonat 30 Minuten bei 50°C gerührt. Anschließend tropft man bei Raumtemperatur 1,5 g (0,015 mol) Butylisocyanat zu, rührt 5 Stunden bei 50°C nach und destilliert das Lösungsmittel ab. Der Rückstand wird in Wasser gelöst und bei 0°C angesäuert. Man extrahiert die Wasserphase mit Dichlormethan, trocknet und dampft ein. Nach Verreiben des Rohprodukts mit Diisopropylether erhält man 3,8 g (74 % d.Th.) 3-Butyl-1-(1-pyrrolidinosulfonylethylsulfonyl)-harnstoff vom Schmp. 153 - 155°C.
b) 1-Pyrrolidinosulfonylethylsulfonylisocyanat
   3,6 g (0,01 mol) 3-Butyl-1-(1-pyrrolidinosulfonylethylsulfonyl)-harnstoff (Beispiel 3a) werden in 70 ml Xylol suspendiert; anschließend wird bei 130°C ca. 2 Stunden Phosgen eingeleitet. Man entfernt das Lösungsmittel im Vakuum und erhält das gewünschte Sulfonylisocyanat in quantitativer Ausbeute (2,7 g).
c) 3-(4-Chlor-6-methylpyrimidin-2-yl)-1-(1-pyrrolidinosulfonylethylsulfonyl)-harnstoff
   Man löst 1,6 g (0,01 mol) 2-Amino-4-chlor-6-methoxypyrimidin in 30 ml Dichlormethan und tropft bei Raumtemperatur 2,7 g (0,01 mol) 1-Pyrrolidinosulfonylethylsulfonylisocyanat (Beispiel 3b) in 30 ml Dichlormethan zu. Nach 3 Stunden Rühren bei Raumtemperatur wäscht man die organische Phase mit 2 N HCl und Wasser, trocknet und dampft ein. Durch Verreiben des Rohprodukts mit Diethylether erhält man 30 g (70 % d. Th.) 3-(4-Chlor-6-methoxypyrimidin-2-yl)-1-(1-pyrrolidinosulfonylethylsulfonyl)-harnstoff vom Schmp. 158 - 160°C.

In analoger Weise werden die in den folgenden Tabellen 1 bis 6 definierten Verbindungen erhalten.

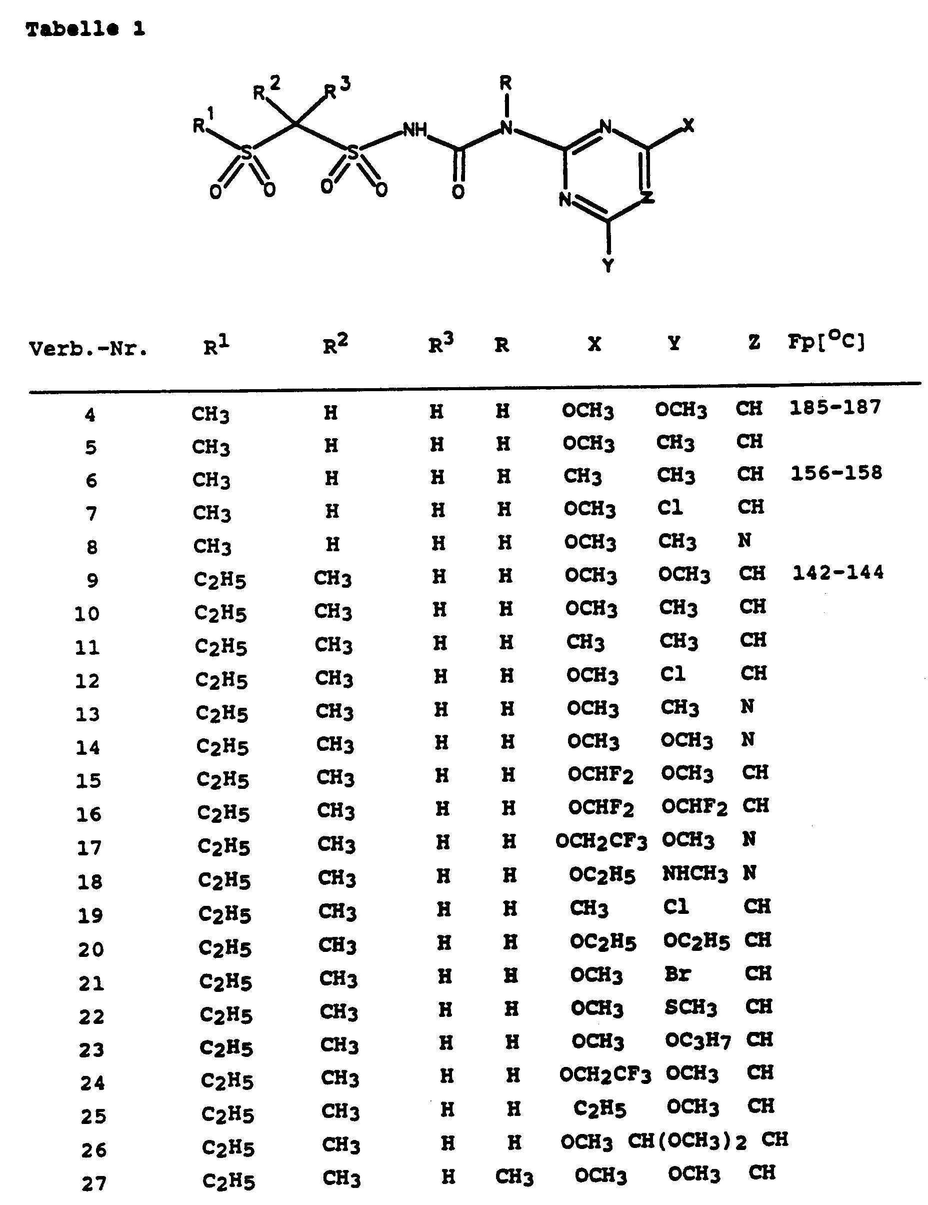

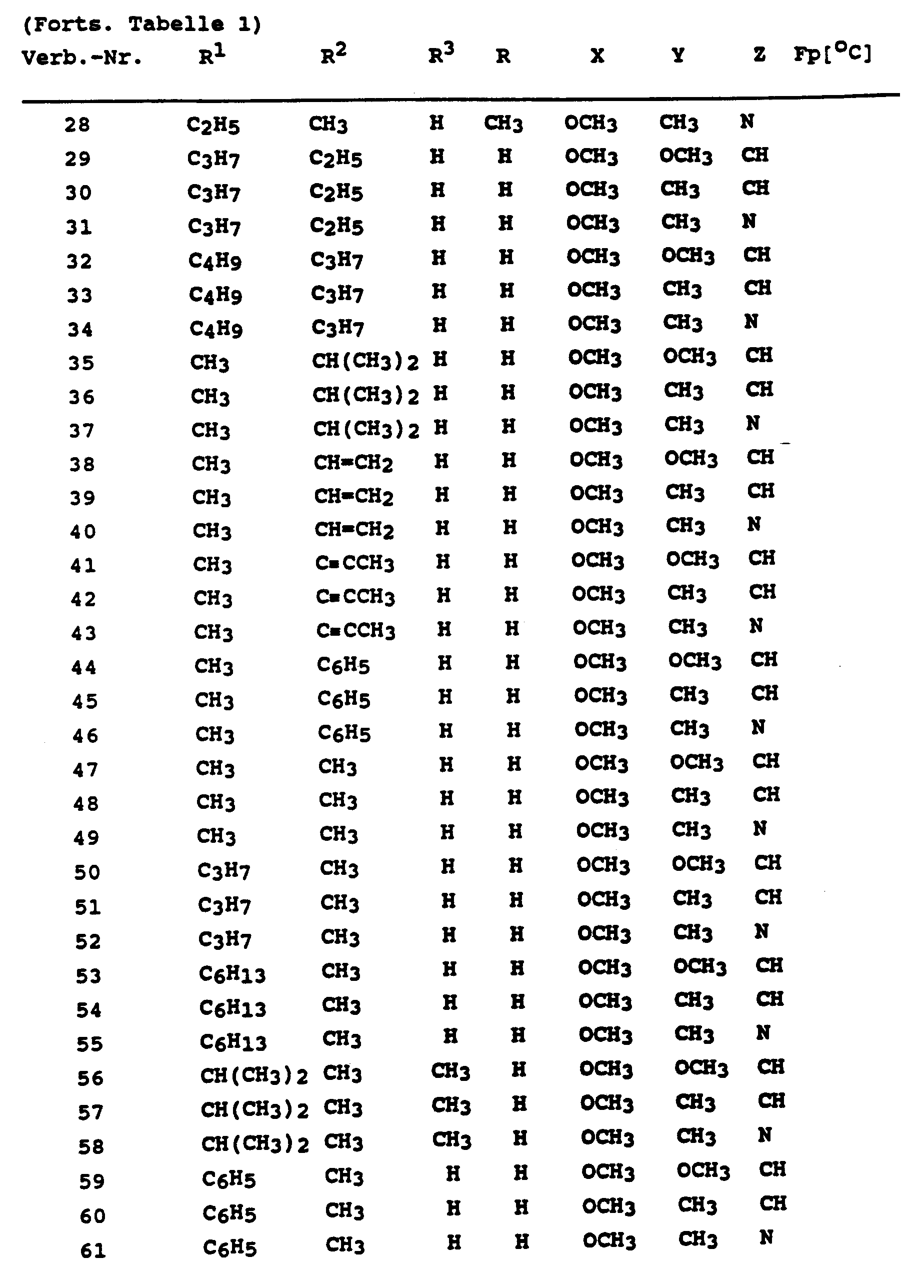

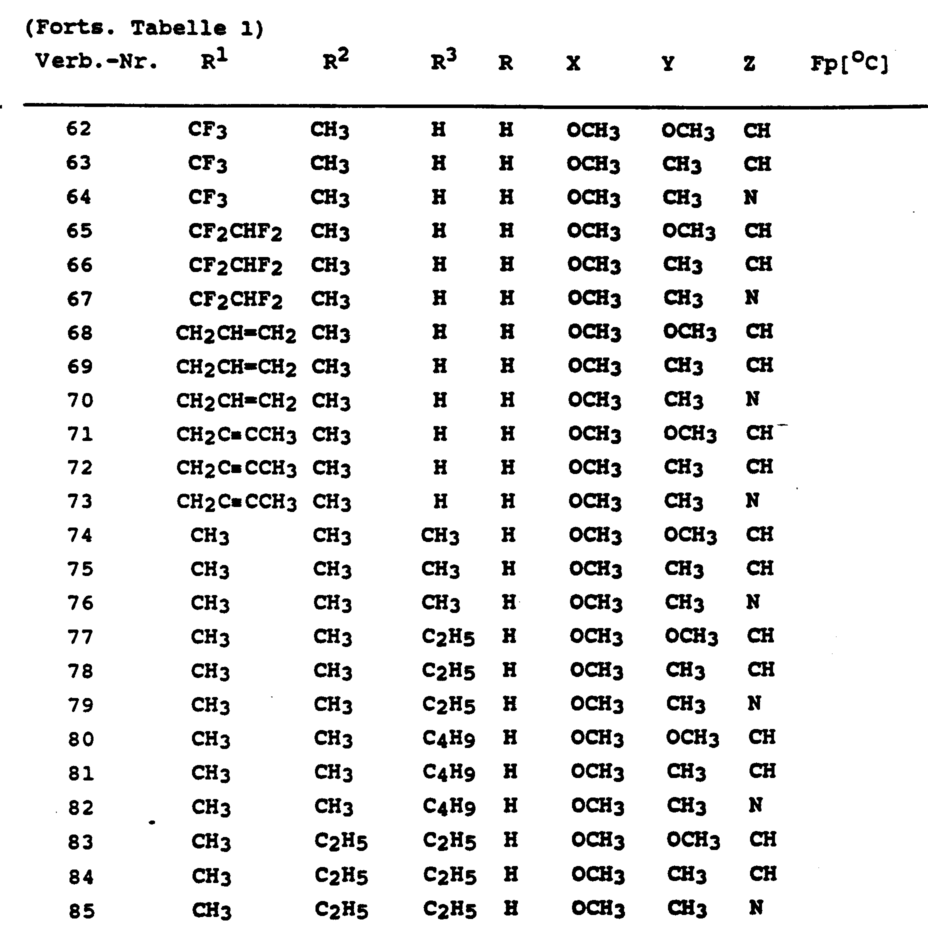

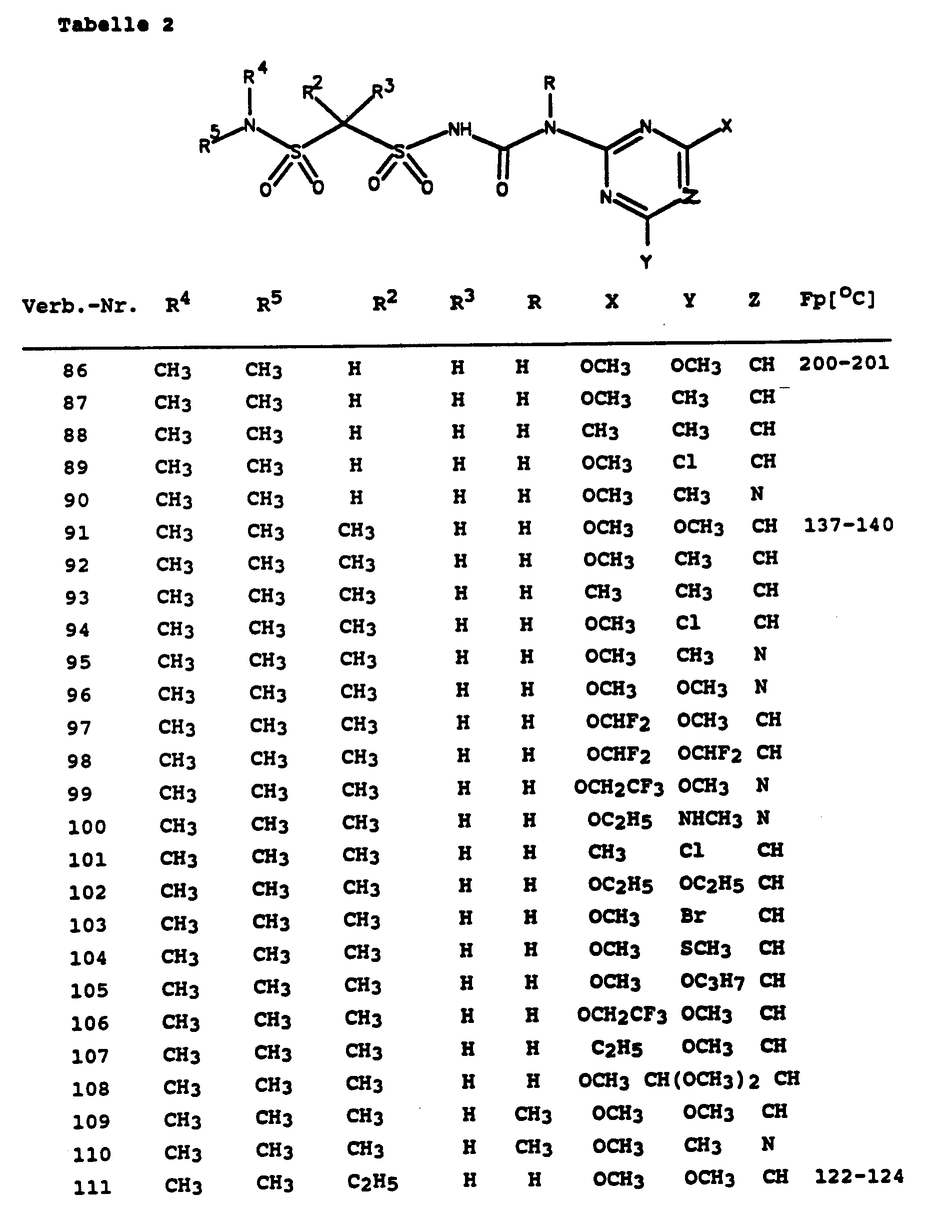

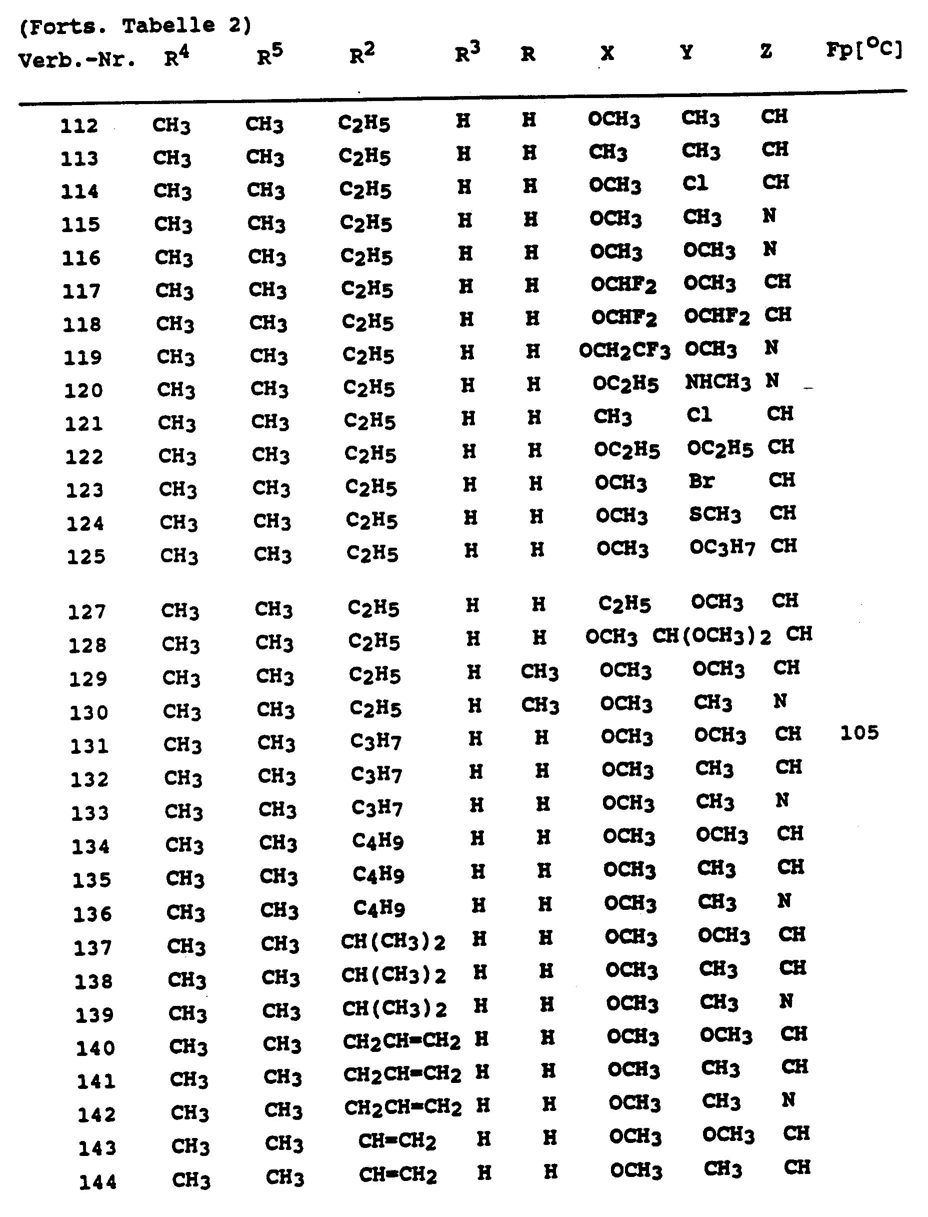

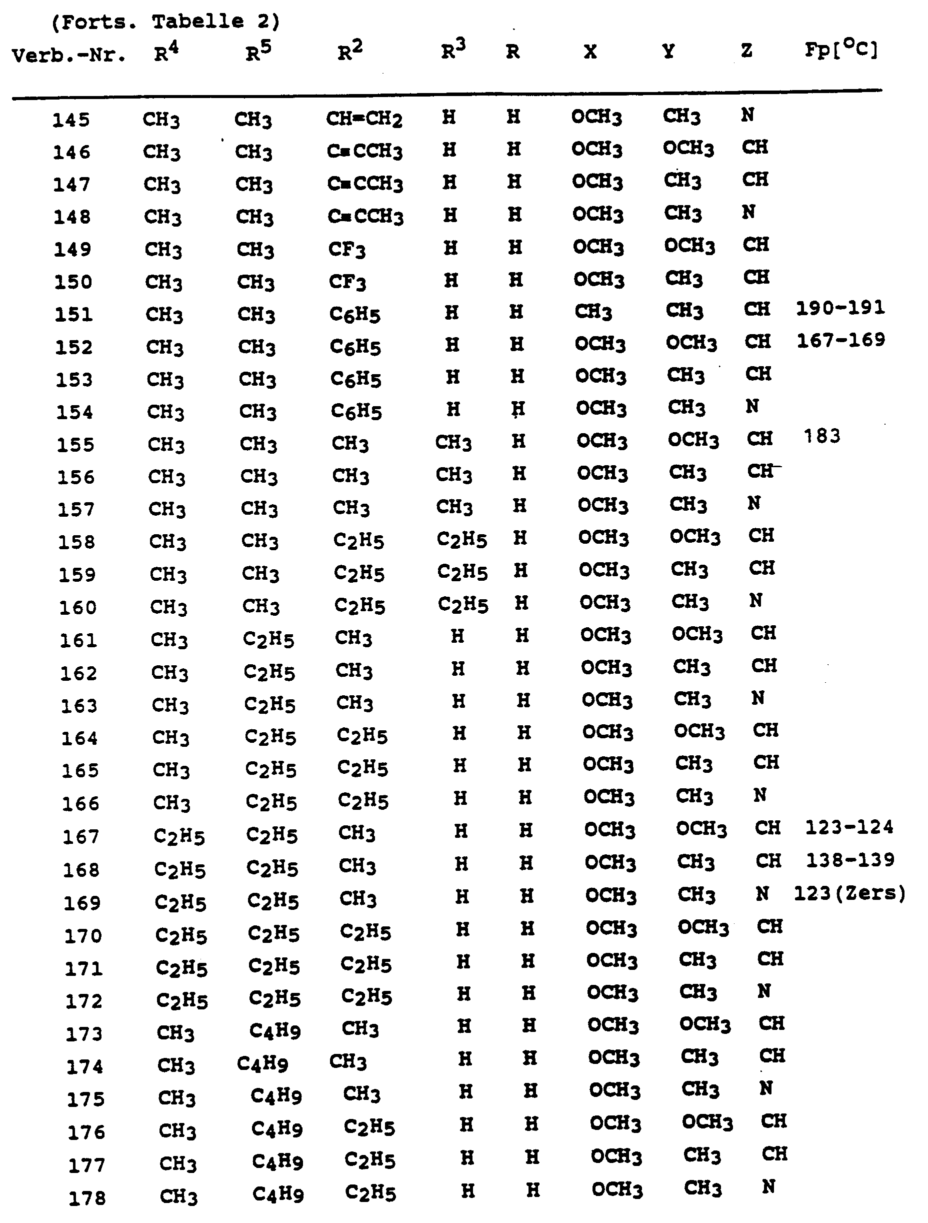

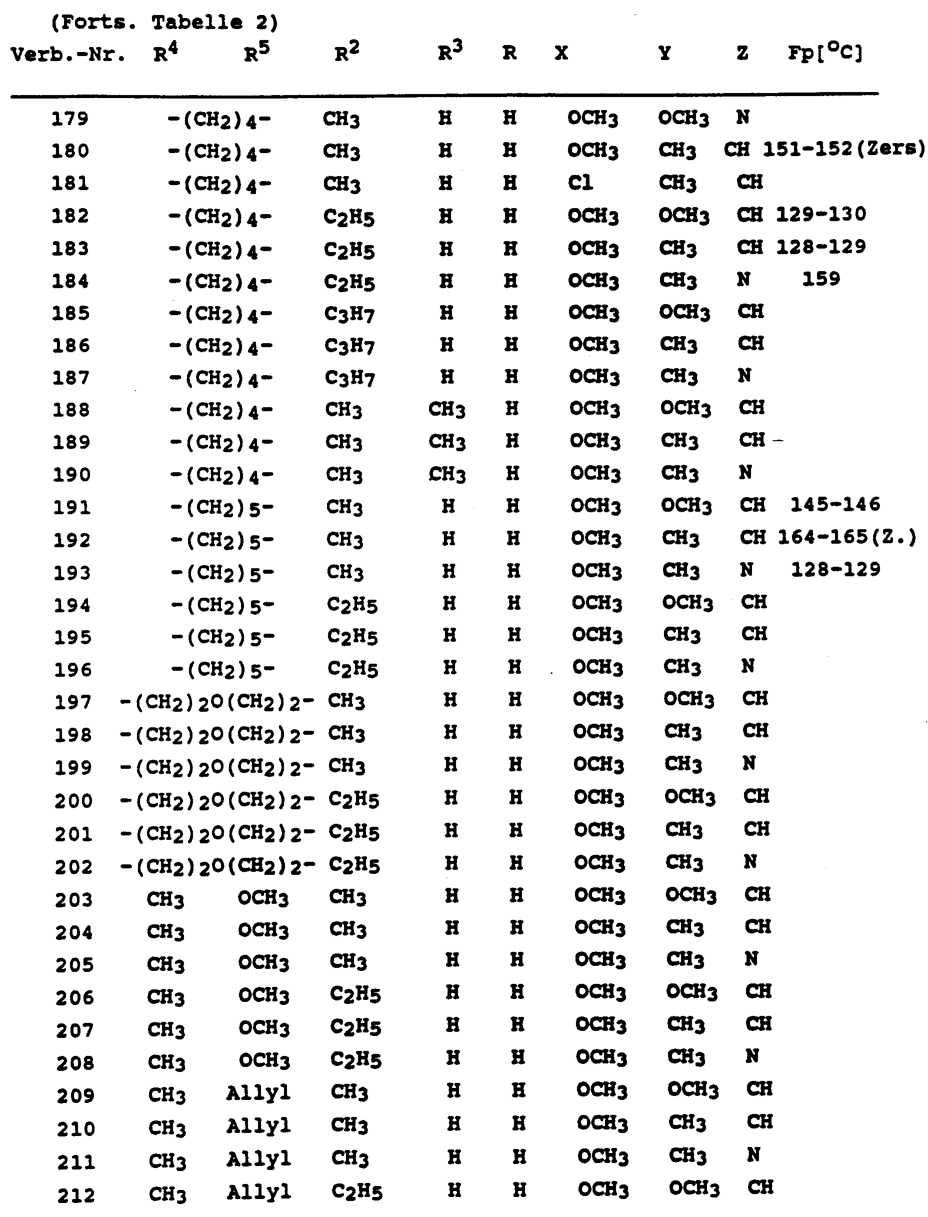

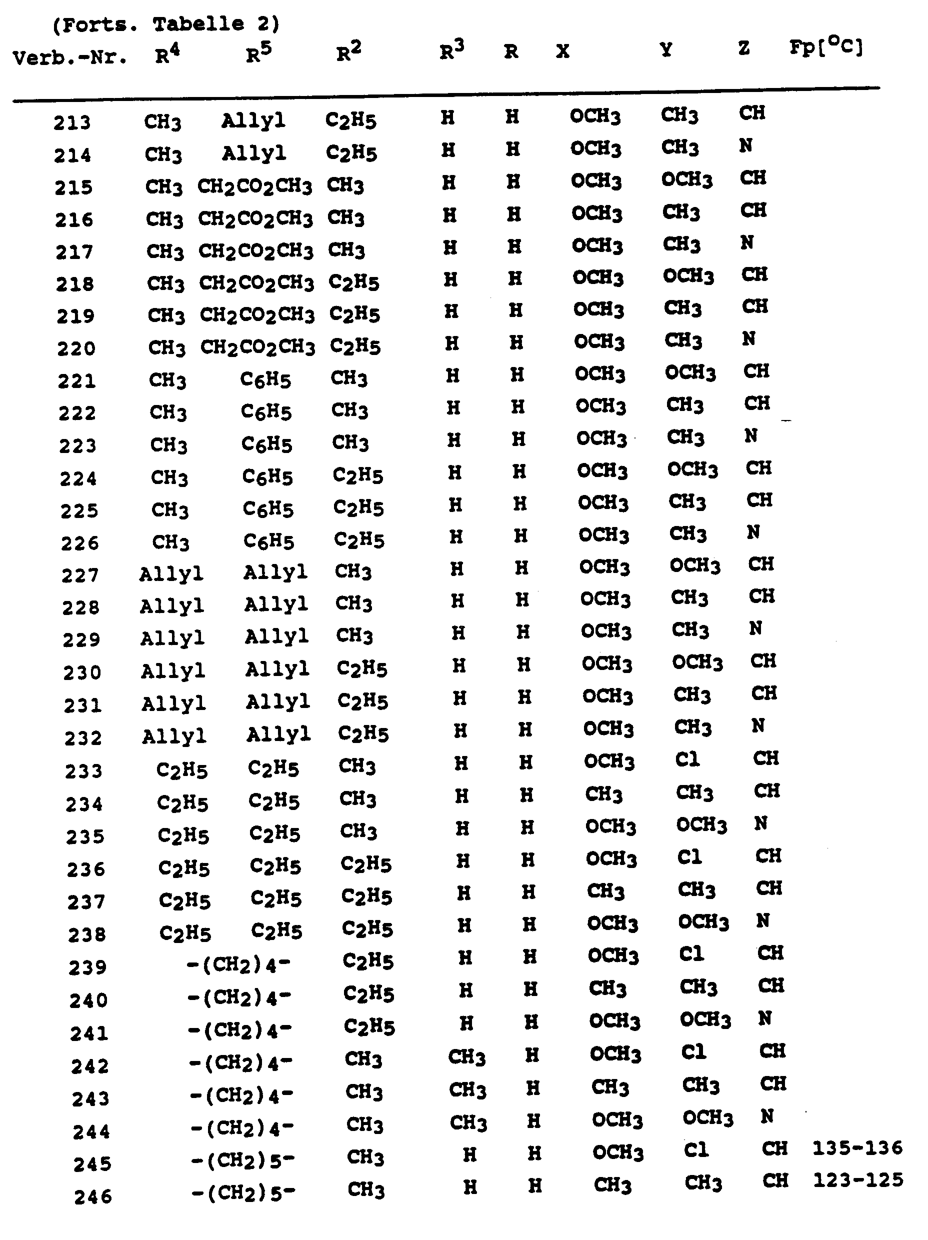

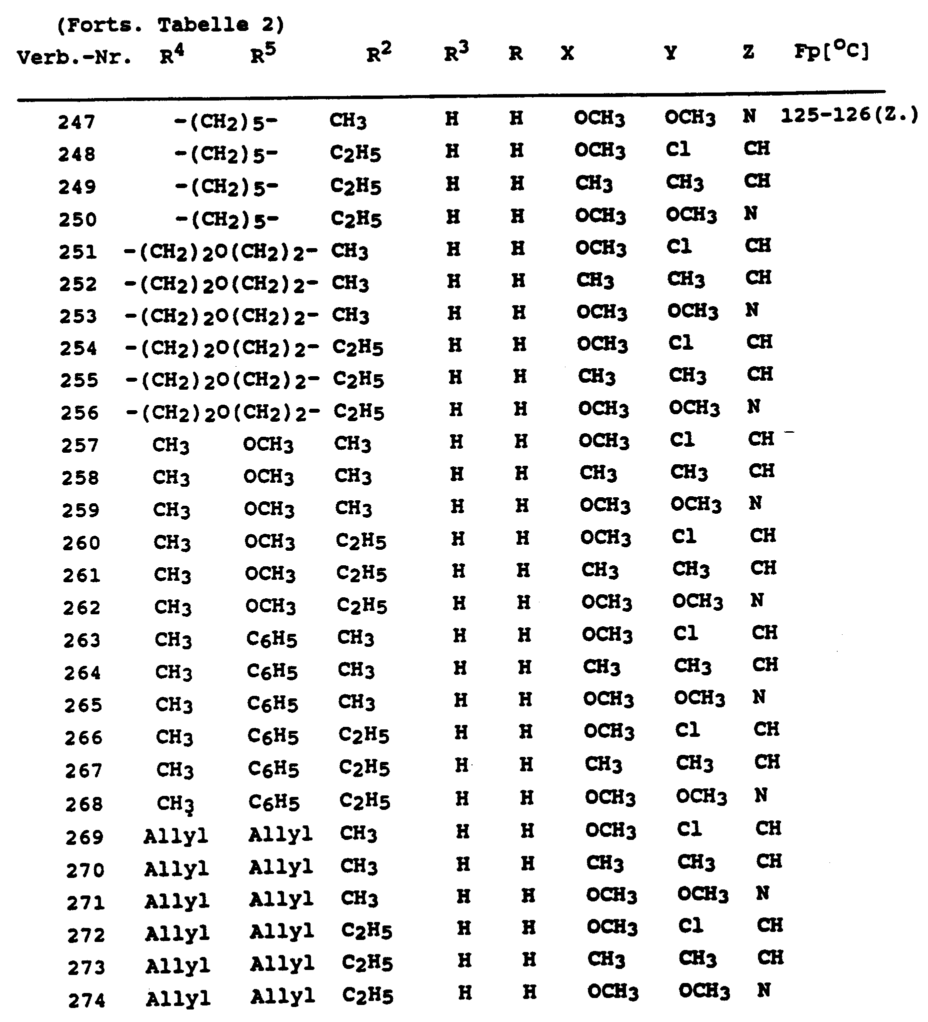

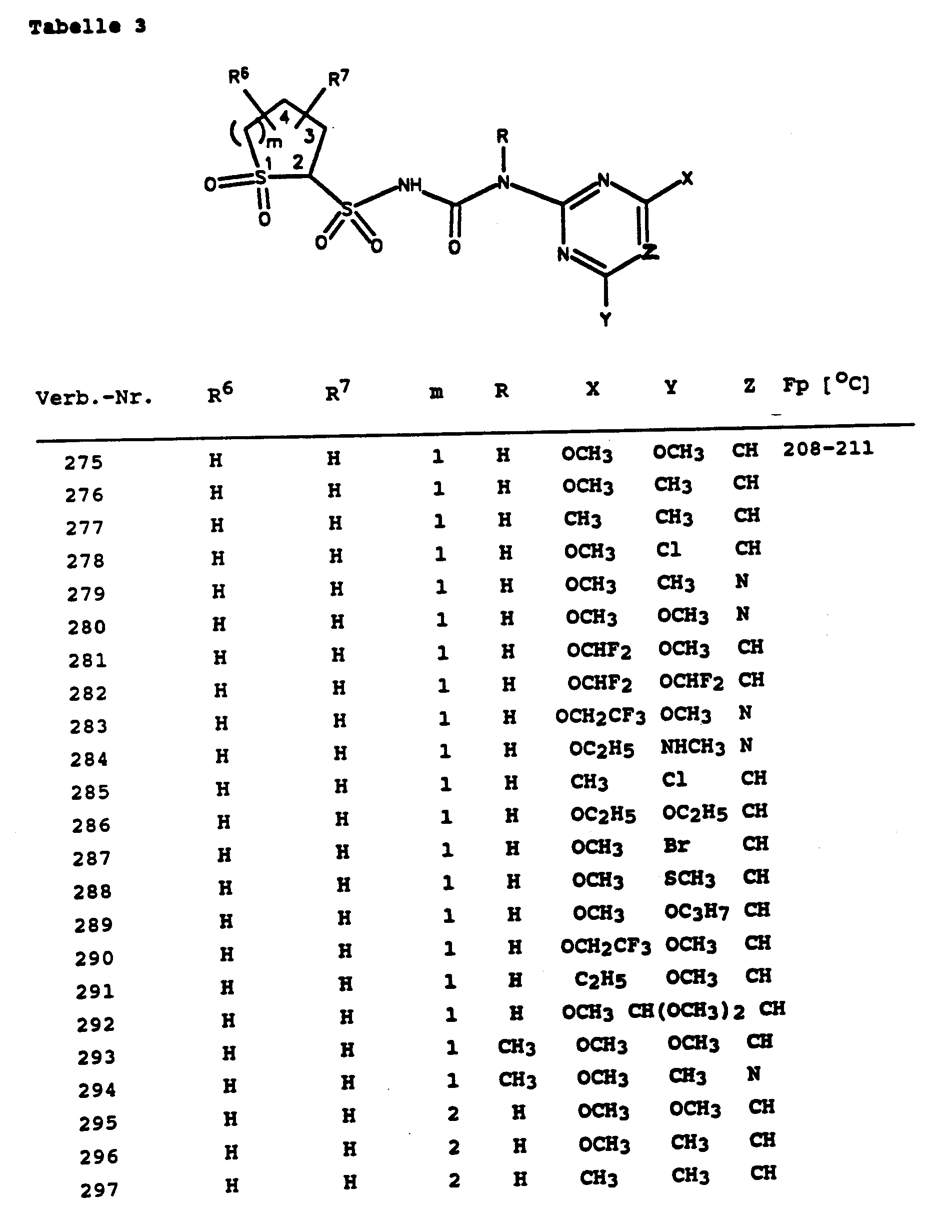

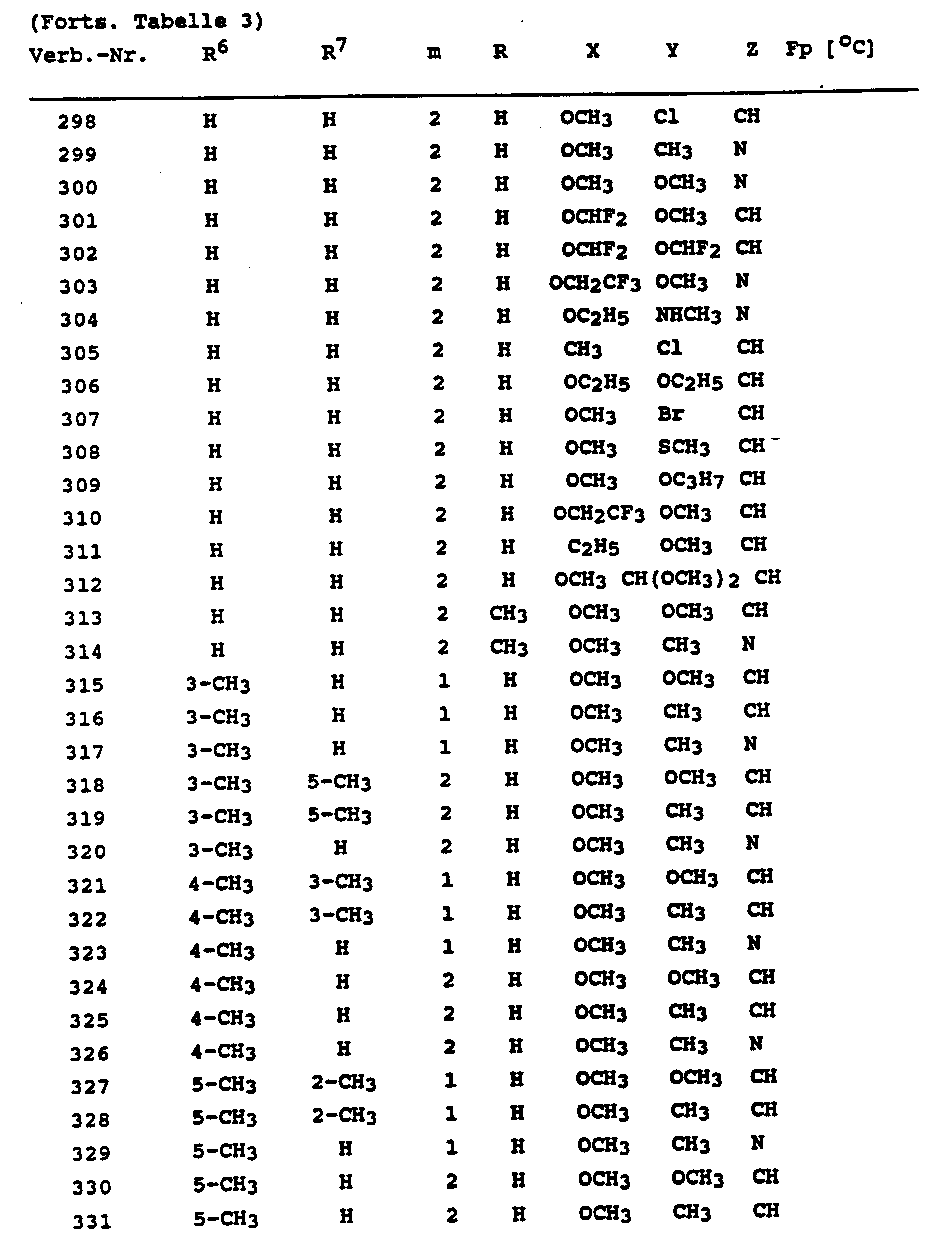

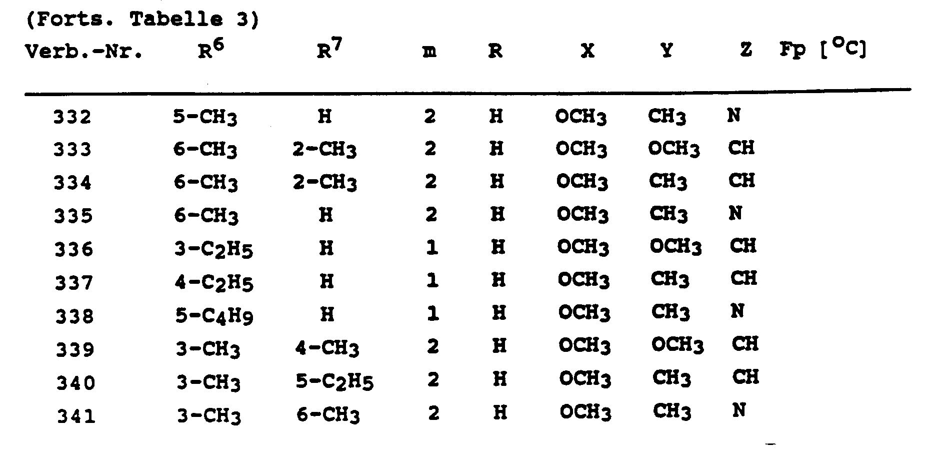

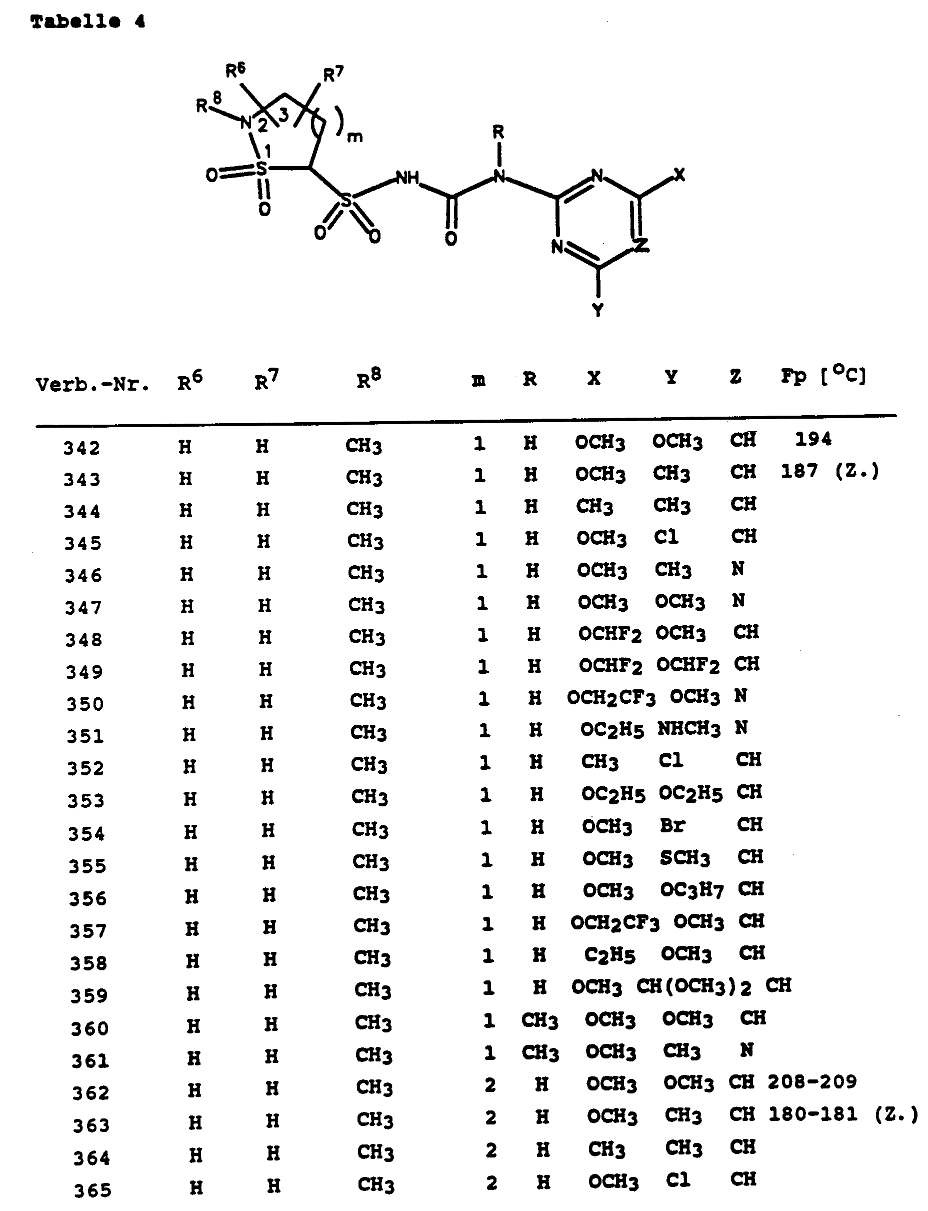

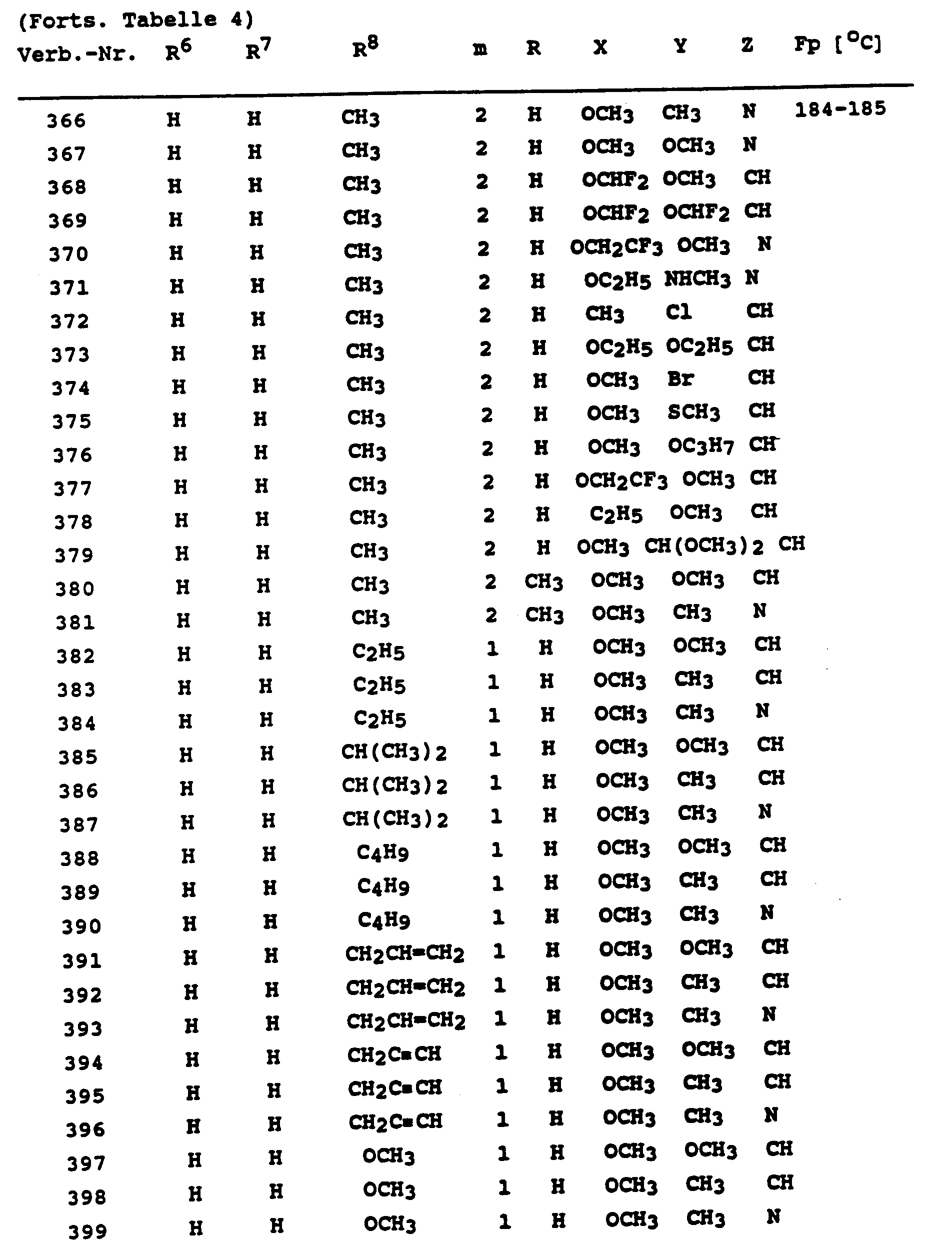

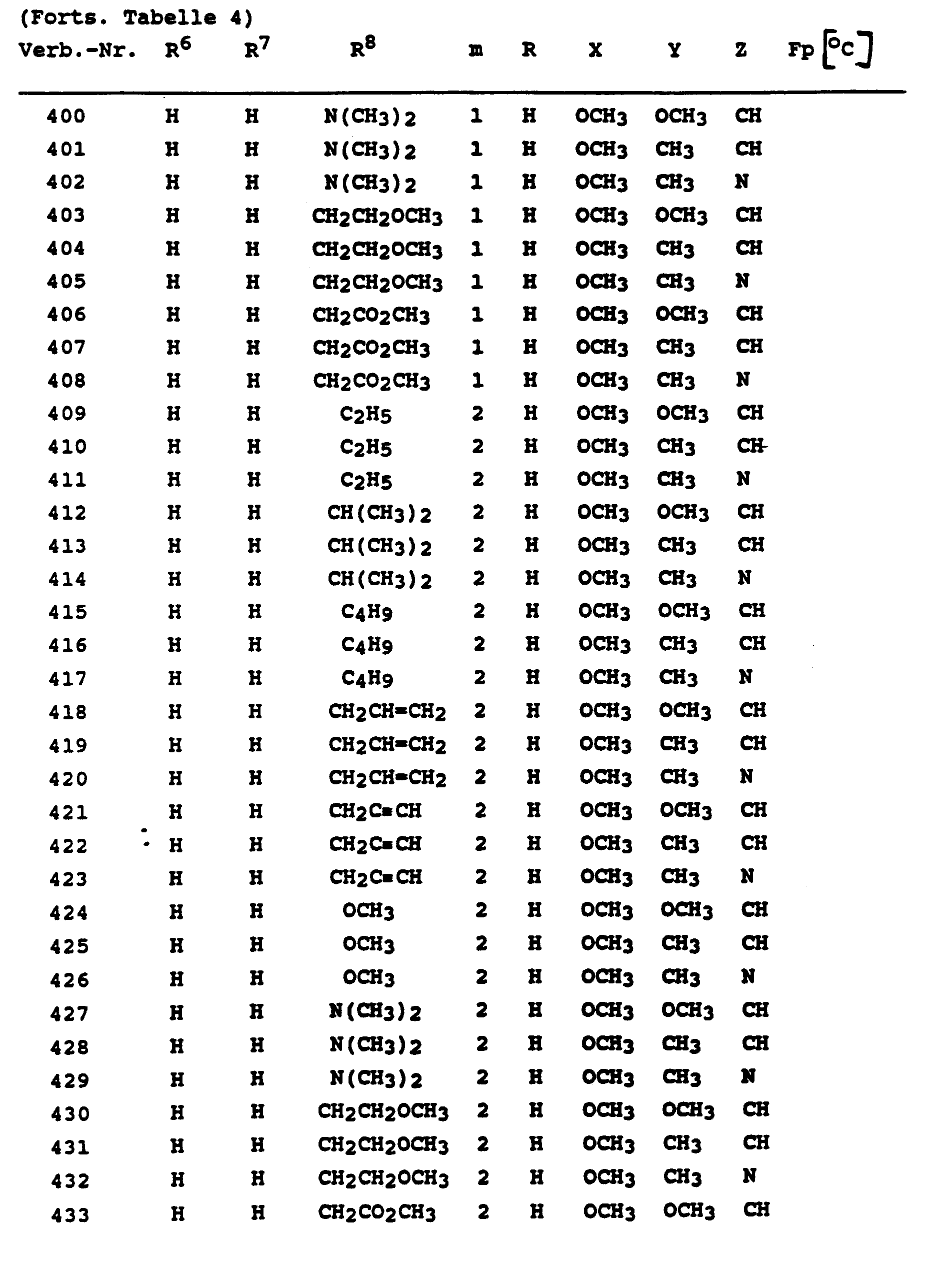

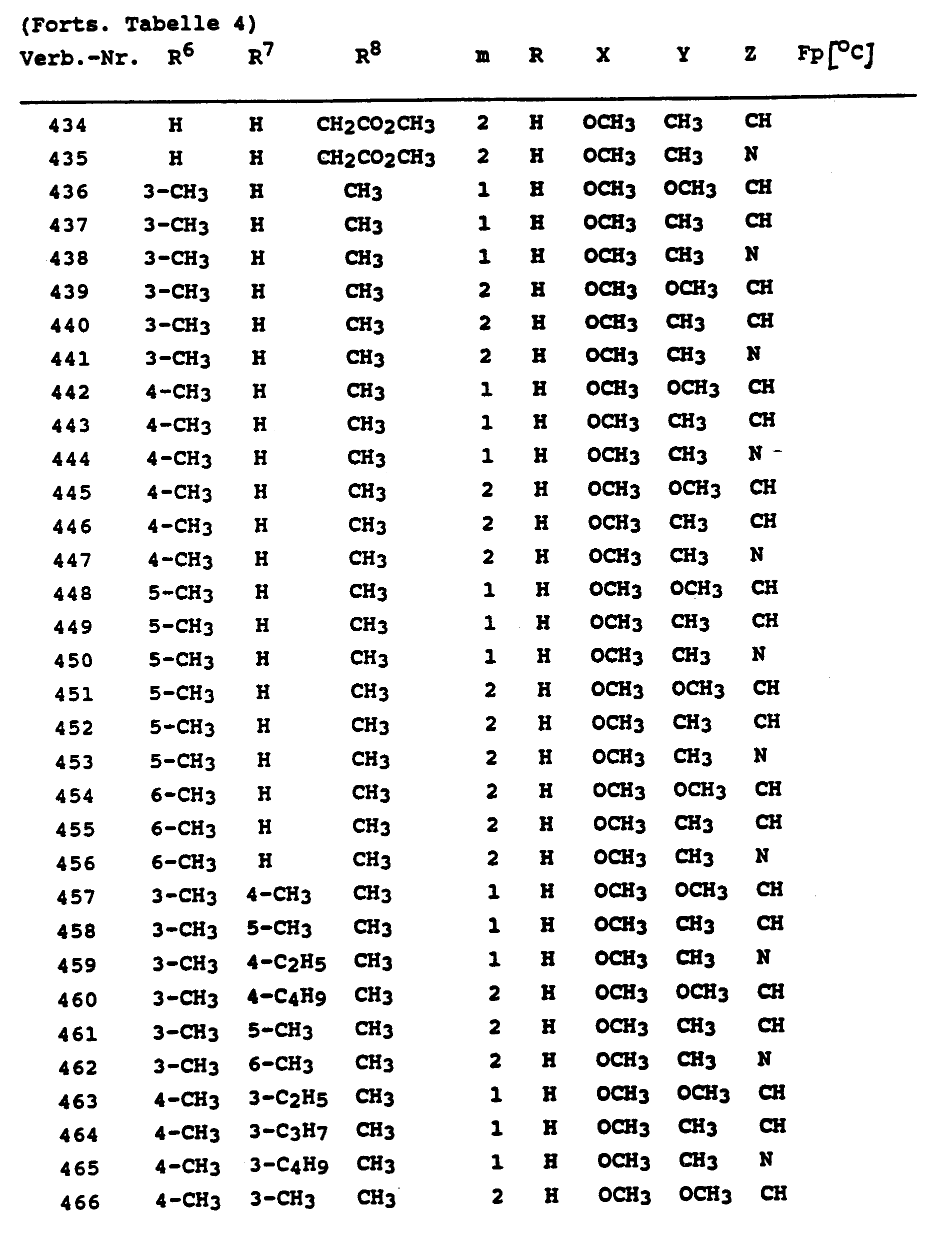

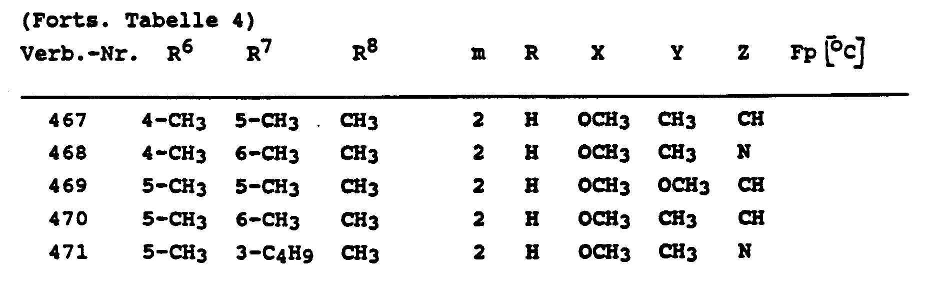

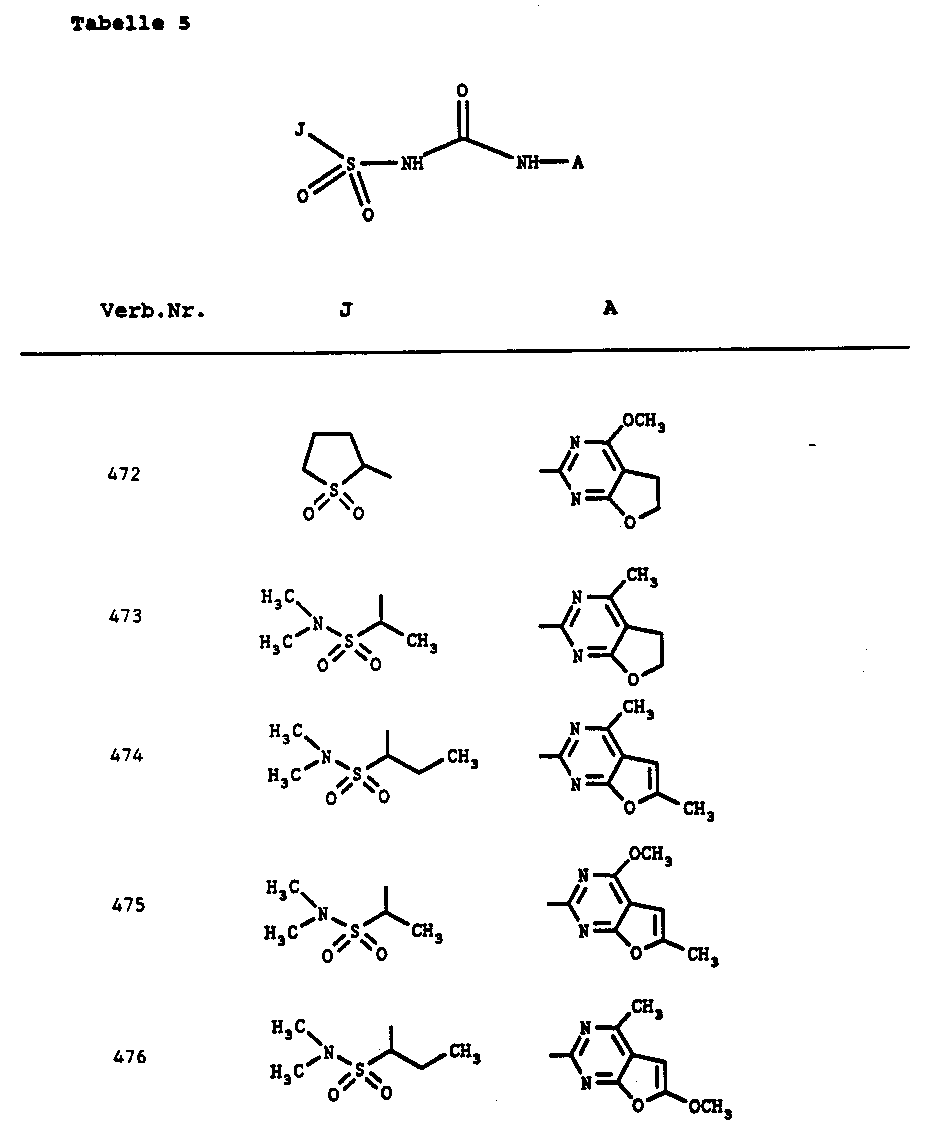

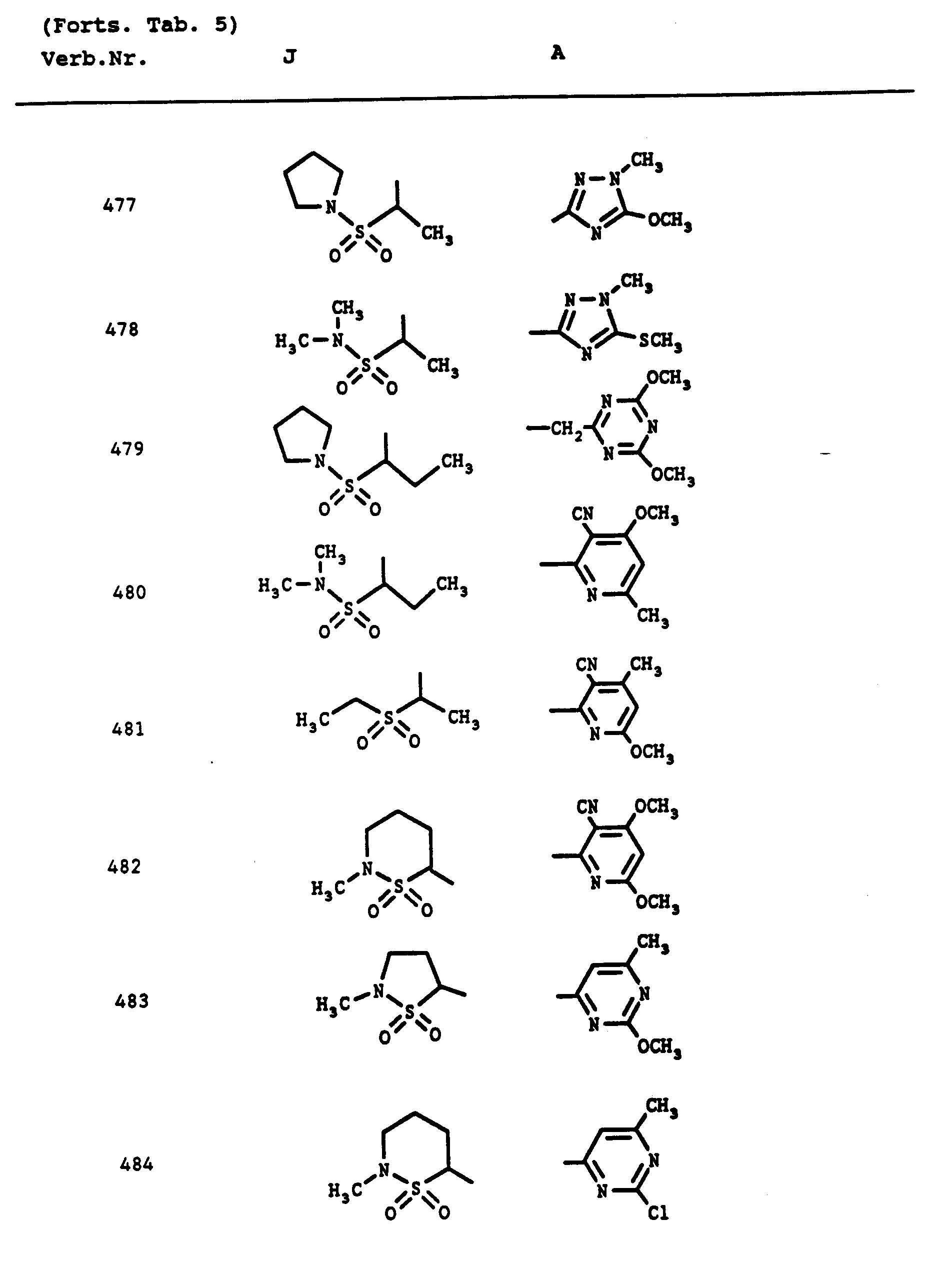

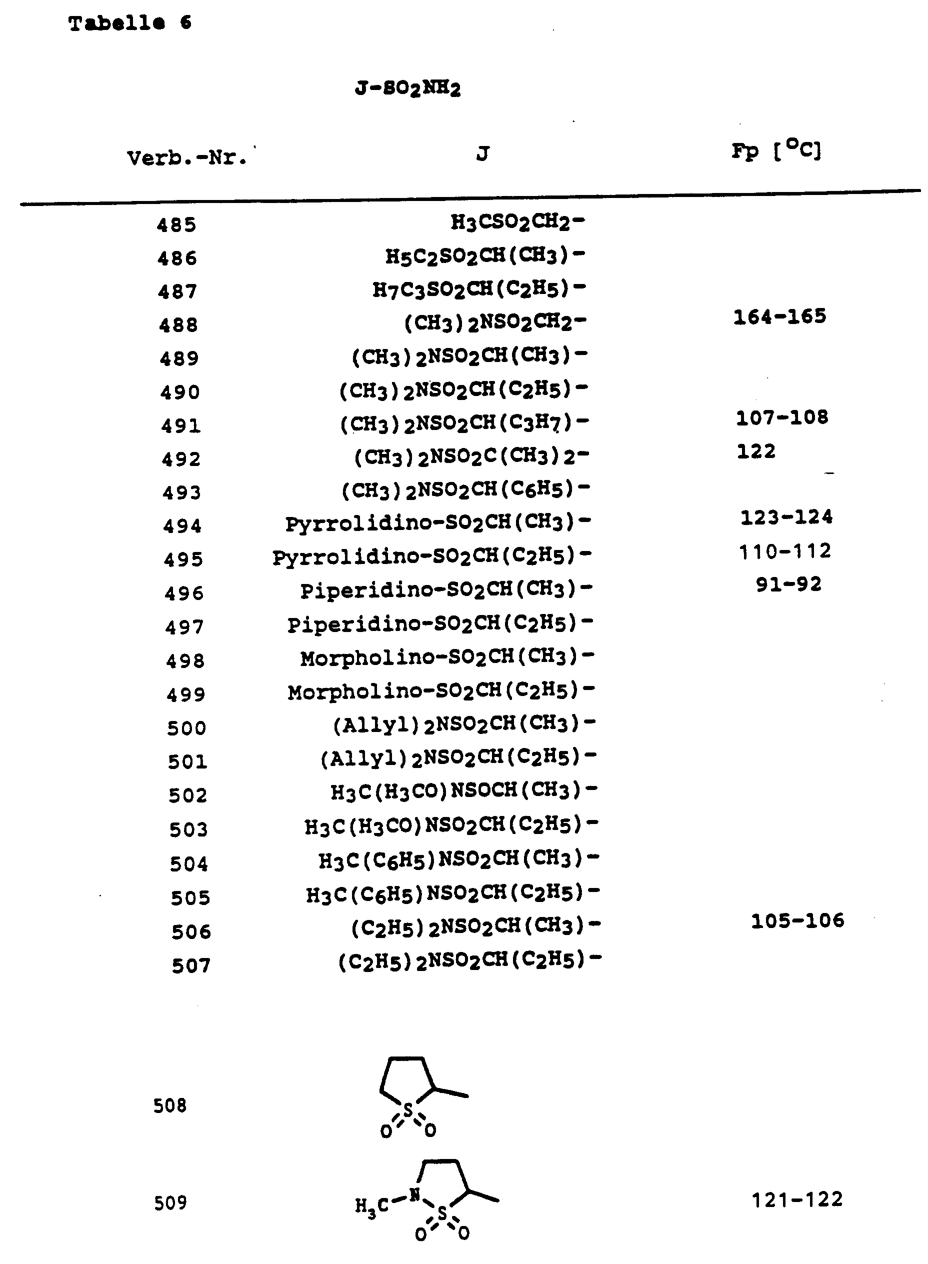

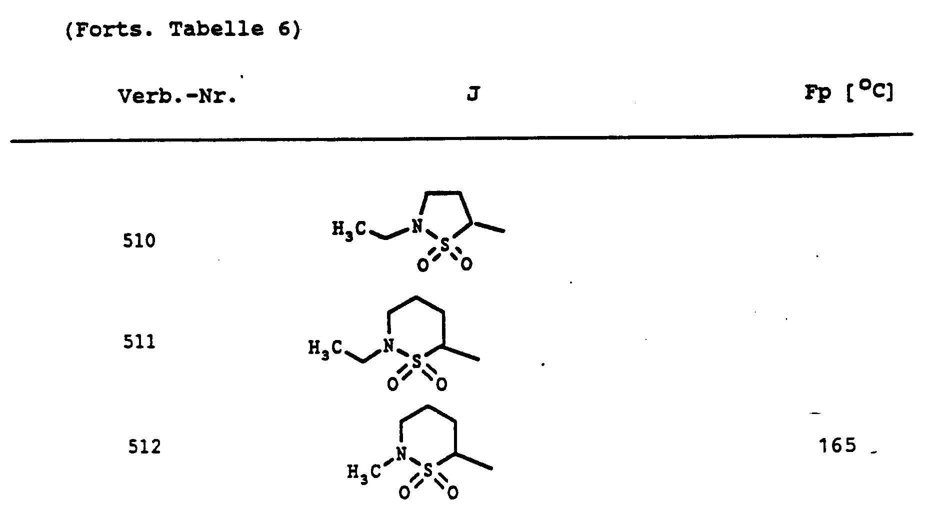

### FORMULIERUNGSBEISPIELE

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inerstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (^{(R)}Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexan als Lösungsmittel und 10 Gew.-Teilen oxethyliertes Nonylphenol als Emulgator.
e) Ein in Wasser dispergierbares Granulat wird erhalten, indem man
   - 75 Gewichtsteile: einer Verbindung der Formel (I),
   - 10 Gewichtsteile: ligninsulfonsaures Calcium,
   - 5 Gewichtsteile: Natriumlaurylsulfat,
   - 3 Gewichtsteile: Polyvinylalkohol und
   - 7 Gewichtsteile: Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   - 25 Gewichtsteile: einer Verbindung der Formel (I),
   - 5 Gewichtsteile: 2,2'-dinaphthylmethan-6,6'-disulfonsaures Natrium,
   - 2 Gewichtsteile: oleoylmethyltaurinsaures Natrium,
   - 1 Gewichtsteile: Polyvinylalkohol,
   - 17 Gewichtsteile: Calciumcarbonat und
   - 50 Gewichtsteile: Wasser
   auf einer Kolloidmühle homogenisiert und vorzerkleinert, anschließend auf einer Perlmüble mahlt und die so erhaltene Suspension in einem Sprühturm mittels einer Einstoffdüse zerstäubt und trocknet.
g) Ein Extruder-Granulat erhält man, indem man 20 Gewichtsteile Wirkstoff, 3 Gewichtsteile ligninsulfonsäures Natrium 1 Gewichtsteil Carboxymethylcellulose und 76 Gewichtsteile Kaolin vermischt, vermahlt und mit Wasser anfeuchtet. Dieses Gemisch wird extrudiert und anschließend in Luftstrom getrocknet.

### BIOLOGISCHE BEISPIELE

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet:
0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte in Tabelle 7 zeigen, weisen die erfindungsgemäßen Verbindungen eine gute herbizide Vorauflaufwirksamkeit gegen ein breites Spektrum von Ungräsern und Unkräutern auf.

**Tabelle 7**

| Vorauflaufwirkung | | | | | |
|---|---|---|---|---|---|
| Beispiel-Nr. | Dosis (kg a.i./ha) | herbizide Wirkung | | | |
| | | SIAL | CRSE | STME | ECCR |
| 4 | 0,3 | 5 | 5 | 4 | 3 |
| 9 | 0,3 | 5 | 5 | 5 | 5 |
| 91 | 0,3 | 5 | 5 | 5 | 5 |
| 111 | 0,3 | 5 | 5 | 5 | 5 |
| Abkürzungen: SIAL = Sinapis alba CRSE = Chrysanthemum segetum STME = Stellaria media ECCR = Echinochloa crus-galli a.i. = Aktivsubstanz | | | | | |

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 8).

**Tabelle 8**

| Nachauflaufwirkung | | | | | |
|---|---|---|---|---|---|
| Beispiel-Nr. | Dosis (kg a.i./ha) | herbizide Wirkung | | | |
| | | SIAL | CRSE | STME | ECCR |
| 4 | 0,3 | 5 | 5 | 4 | 4 |
| 9 | 0,3 | 4 | 5 | 4 | 4 |
| 91 | 0,3 | 5 | 5 | 4 | 4 |
| 111 | 0,3 | 5 | 5 | 5 | 5 |

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) weisen somit eine hohe Selektivität bei Anwendung zur Bekämpfung von unerwünschtem Pflanzenwuchs in landwirtschaftlichen Kulturen auf.

## Patentansprüche

1. Verbindungen der allgemeinen Formel (I) oder deren Salze 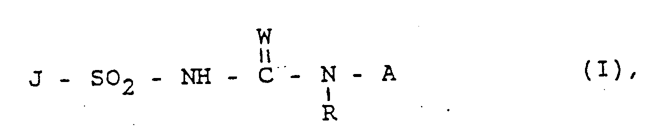 worin J einen Rest der Formel (J-1)-(J-4) 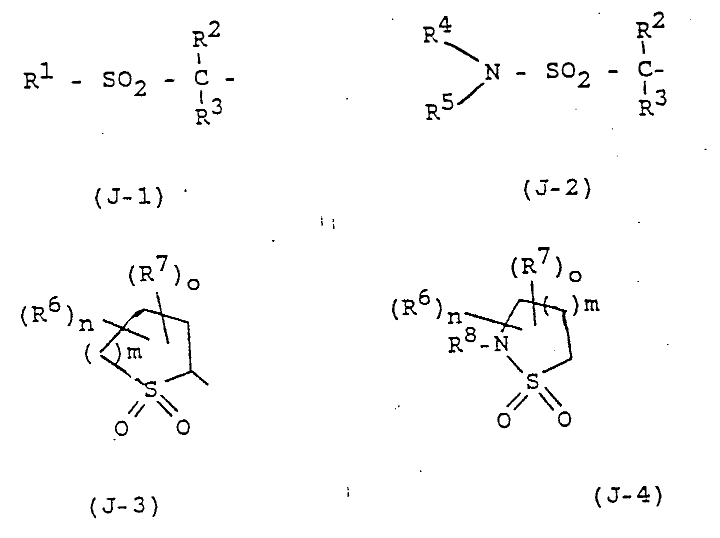 und darin
R¹ (C₁-C₆)Alkyl, (C₂-C₆)Alkenyl, (C₂-C₆)Alkinyl, wobei die letztgenannten 3 Reste unsubstituiert oder ein- oder mehrfach durch Halogen oder ein- oder zweifach durch Reste aus der Gruppe bestehend aus (C₁-C₄)Alkoxy, (C₁-C₄)Alkylthio und (C₁-C₄)Alkoxycarbonyl substituiert sind, oder -(CH₂)ₐ-Phenyl, wobei a 0,1 oder 2 bedeutet und der Phenylrest unsubstituiert oder ein- oder mehrfach durch Reste aus der Gruppe Halogen, (C₁-C₄)Alkyl, (C₁-C₄)Alkoxy, (C₁-C₄)Alkylthio, (C₁-C₄)Alkylsulfinyl, (C₁-C₄)Alkylsulfonyl und (C₁-C₄-Alkoxy-carbonyl und Reste analog letztgenannten 6 Resten, die im Alkylteil ein- oder mehrfach halogeniert sind, substituiert ist,
R²,R³ unabhängig voneinander Wasserstoff, Halogen, (C₁-C₄)Alkoxy, (C₁-C₄)Alkylthio, (C₁-C₆)Alkyl, (C₂-C₆)Alkenyl, (C₂-C₆)Alkinyl, wobei die letztgenannten 5 Reste unsubstituiert oder ein- oder mehrfach durch Halogen oder ein- oder zweifach durch Reste aus der Gruppe (C₁-C₄)Alkoxy, (C₁-C₄)Alkylthio substituiert sind, (C₁-C₄-Alkoxy)-carbonyl-(C₁-C₃)-alkyl, -(CH₂)ₐ-Phenyl, wobei a 0, 1 oder 2 bedeutet und der Phenylrest unsubstituiert oder durch ein oder mehrere Reste aus der Gruppe Halogen, (C₁-C₄)Alkyl, (C₁-C₄)Alkoxy, (C₁-C₄-Alkoxy)-carbonyl, (C₁-C₄)Alkylthio und (C₁-C₄)Alkylsulfonyl, wobei die letztgenannten 5 Reste im Alkylteil unsubstituiert oder ein- oder mehrfach durch Halogen substituiert sind, substituiert ist,
R⁴,R⁵ unabhängig voneinander Wasserstoff, (C₁-C₈)Alkyl, (C₂-C₈)Alkenyl, (C₂-C₈)Alkinyl, wobei die 3 letztgenannten Reste unsubstituiert oder ein- oder mehrfach durch Halogen oder ein- oder zweifach durch (C₁-C₄)Alkoxy oder (C₁-C₄)-Alkylthio substituiert sind, (C₁-C₄)Alkoxycarbonyl-(C₁-C₃)alkyl, -(CH₂)ₐ-Phenyl, wobei a 0, 1 oder 2 bedeutet und der Phenylrest unsubstituiert oder ein- oder mehrfach durch Reste aus der Gruppe Halogen, (C₁-C₄)Alkyl, (C₁-C₄)Alkoxy, (C₁-C₄)Alkylthio, (C₁-C₄)Alkylsulfonyl, wobei die vorgenannten 4 Reste im Alkylteil unsubstituiert oder ein- oder mehrfach durch Halogen substituiert sind, und (C₁-C₄-Alkoxy)-carbonyl substituiert ist, (C₁-C₆)Alkoxy oder Di-(C₁-C₆)alkylamino oder R⁴ und R⁵ zusammen mit dem sie verknüpfenden N-Atom einen heterocyclischen Ring der Formel 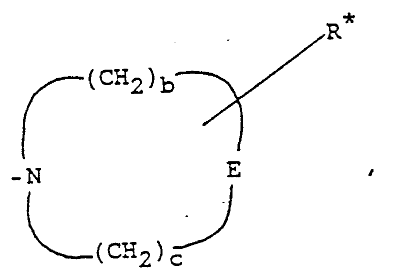 wobei b und c unabhängig voneinander 0, 1, 2 oder 3, R* Wasserstoff, (C₁-C₄)Alkyl, (C₁-C₄-Alkoxy)-carbonyl oder (C₁-C₄-Alkoxy)-methyl und E eine divalente Gruppe der Formel O, S, CH₂ oder N-(C₁-C₄)Alkyl bedeuten,
R⁶,R⁷ unabhängig voneinander Halogen, (C₁-C₈)Alkyl, (C₂-C₈)Alkenyl, (C₂-C₈)Alkinyl oder (C₁-C₈)Alkoxy, wobei die letztgenannten 4 Reste unsubstituiert oder ein- oder mehrfach durch Halogen oder ein- oder zweifach durch (C₁-C₄)Alkoxy oder (C₁-C₄)Alkylthio substituiert sind, oder (C₁-C₄-Alkoxy)-carbonyl-(C₁-C₃)alkyl,
R⁸ Wasserstoff, (C₁-C₈)Alkyl, (C₂-C₈)Alkenyl, (C₂-C₈)Alkinyl oder (C₁-C₈)Alkoxy, wobei die letztgenannten 4 Reste unsubstituiert oder ein- oder mehrfach durch Halogen oder ein- oder zweifach durch (C₁-C₄)Alkoxy oder (C₁-C₄)-Alkylthio substituiert sind, oder (C₁-C₄-Alkoxy)-carbonyl-(C₁-C₃)alkyl,
R⁹ Wasserstoff, (C₁-C₈)Alkyl, (C₂-C₈)Alkenyl, (C₂-C₈)Alkinyl oder (C₁-C₈)Alkoxy, wobei die letztgenannten 4 Reste unsubstituiert oder ein- oder mehrfach durch Halogen oder ein- oder zweifach durch (C₁-C₄)Alkoxy oder (C₁-C₄)-Alkylthio substituiert sind, oder (C₁-C₄-Alkoxy)-carbonyl-(C₁-C₃)alkyl,
R¹⁰ Wasserstoff, (C₁-C₄)Alkyl, (C₁-C₄-Alkoxy)-carbonyl oder (C₁-C₄-Alkoxy)-methyl,
m 1, 2 oder 3,
n,o unabhängig voneinander 0, 1, 2 oder 3,
A einen Rest der Formeln (A-1) bis (A-8) 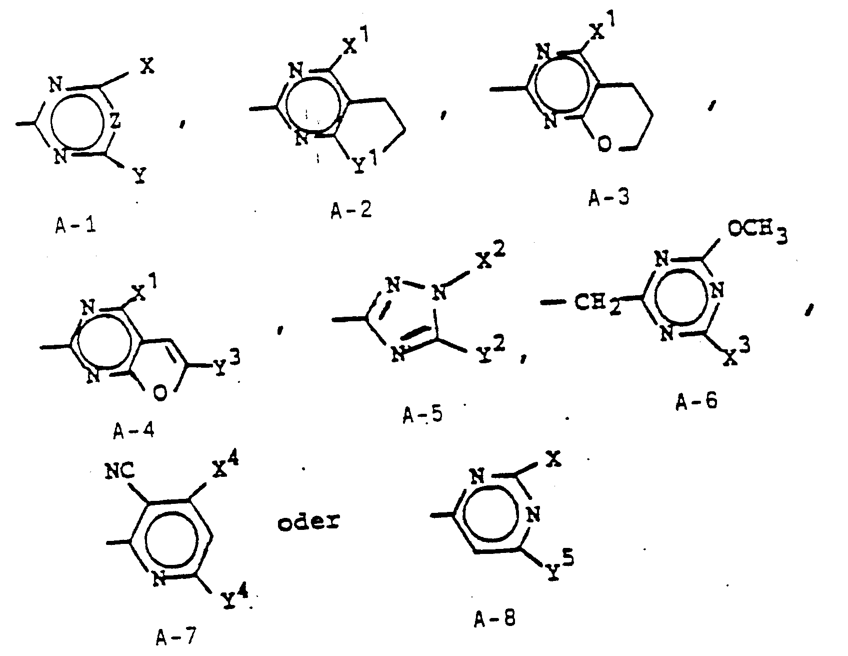
X, Y unabhängig voneinander H, Halogen, (C₁-C₃)Alkyl, (C₁-C₃)Alkoxy oder (C₁-C₃)Alkylthio, wobei die vorgenannten alkylhaltigen Reste ein- oder mehrfach durch Halogen oder ein- oder zweifach durch (C₁-C₃)Alkoxy oder (C₁-C₃)Alkylthio substituiert sein können, ferner einen Rest der Formel NR⁹R¹⁰, (C₃-C₆)Cycloalkyl, (C₂-C₄)Alkenyl, (C₂-C₄)Alkinyl, (C₃-C₄)Alkenyloxy oder (C₃-C₄)Alkinyloxy,
Z CH oder N,
X¹ CH₃, OCH₃, OC₂H₅ oder OCF₂H,
Y¹ -O- oder -CH₂-,
X² CH₃, C₂H₅ oder CH₂CF₃,
Y² OCH₃, OC₂H₅, SCH₅, SC₂H₅, CH₃ oder C₂H₅,
X³ CH₃ oder OCH₃,
Y³ H oder CH₃,
X⁴ CH₃, OCH₃, OC₂H₅, CH₂OCH₃ oder Cl,
Y⁴ CH₃, OCH₃, OC₂H₅ oder Cl,
Y⁵ CH₃, C₂H₅, OCH₃ oder Cl,
W O oder S und
R Wasserstoff oder CH₃
bedeuten.

2. Verbindungen und deren Salze nach Anspruch 1, dadurch gekennzeichnet, daß A einen Rest der Formel (A-1) bedeutet.

3. Verbindungen und deren Salze nach Anspruch 1 oder 2, dadurch gekennzeichnet, daß
R¹ = (C₁-C₄)Alkyl, R² und R³ unabhängig voneinander Wasserstoff, (C₁-C₄)Alkyl oder (C₂-C₄)Alkenyl, R⁴ und R⁵ unabhängig voneinander Wasserstoff, (C₁-C₄)Alkyl, (C₁-C₄)Alkoxy, (C₂-C₄)Alkenyl oder R⁴ und R⁵ zusammen einen heterocyclischen Rest der Formel 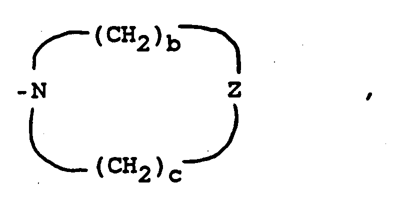 wobei b und c unabhängig voneinander 0, 1, 2 oder 3, die Summe von b+c die Zahl 3 oder 4 und Z O oder CH₂ sind, R⁶ und R⁷ unabhängig voneinander (C₁-C₃)Alkyl, R⁸ (C₁-C₄)Alkyl oder (C₂-C₄)Alkenyl, R⁹ und R¹⁰ unabhängig voneinander Wasserstoff oder (C₁-C₄)Alkyl, m 1 oder 2, n und o unabhängig voneinander 0, 1 oder 2, vorzugsweise 0 oder 1, A einen Rest der Formel (A-1), X und Y unabhängig voneinander Halogen, (C₁-C₄)Alkyl oder (C₁-C₄)Alkoxy, wobei die beiden letztgenannten Reste ein- oder mehrfach durch Halogen substituiert sein können, und W ein Sauerstoffatom bedeuten.

4. Verfahren zur Herstellung von Verbindungen der Formel (I) oder deren Salze nach einem oder mehreren der Ansprüchen 1 bis 3, dadurch gekennzeichnet, daß man
a) eine Verbindung der Formel (II),
J - SO₂NH₂ (II)
worin J wie in Formel (I) definiert ist, mit einem heterocyclischen (Thio)-Carbamat der Formel (III) umsetzt, 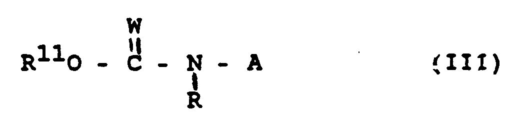 wobei R¹¹ = (C₁-C₆)Alkyl, (C₁-C₄)Halogenalkyl oder Phenyl, das unsubstituiert oder ein- oder mehrfach durch Reste aus der Gruppe Halogen, (C₁-C₄)Alkyl und NO₂ substituiert ist,
bedeutet, und A, R und W die Bedeutungen wie bei Formel (I) haben,
b) ein Sulfonylcarbamat der Formel (IV), 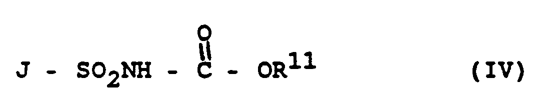 worin R¹¹ wie unter a) definiert ist,
mit einem Aminoheterocyclus der Formel (V), 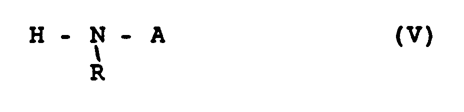 in welcher A und R wie in Formel (I) definiert sind, oder
c) ein Sulfonylisocyanat der Formel (VI),
J - SO₂NCO (VI)
in welcher J wie in Formel (I) definiert ist,
mit einem Aminoheterocyclus der Formel (V) umsetzt.

5. Herbizide oder pflanzenwachstumsregulierende Mittel, dadurch gekennzeichnet, daß sie eine Verbindung der Formel (I) oder ihr Salz nach einem oder mehreren der Ansprüche 1 bis 3 neben üblichen Formulierungsbilfsmitteln enthalten.

6. Verwendung von Verbindungen der Formel (I) oder deren Salzen nach einem oder mehreren der Ansprüche 1 bis 3 als Herbizide oder Pflanzenwachstumsregulatoren.

7. Verfahren zur selektiven Bekämpfung von Schadpflanzen oder zur Wachstumsregulierung von Pflanzen, dadurch gekennzeichnet, daß man eine wirksame Menge einer nach einem oder mehreren der Ansprüche 1 bis 3 definierten Verbindung oder deren Salze auf die Pflanzen, Pflanzensamen oder deren Anbaufläche appliziert.

## Claims

1. A compound of the formula (I) or salts thereof 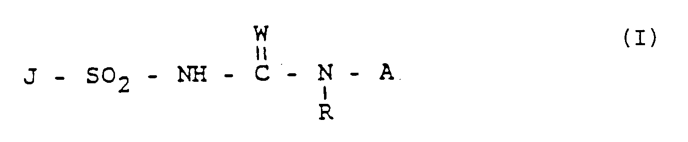 in which J is a radical of the formula (J-1)-(J-4) 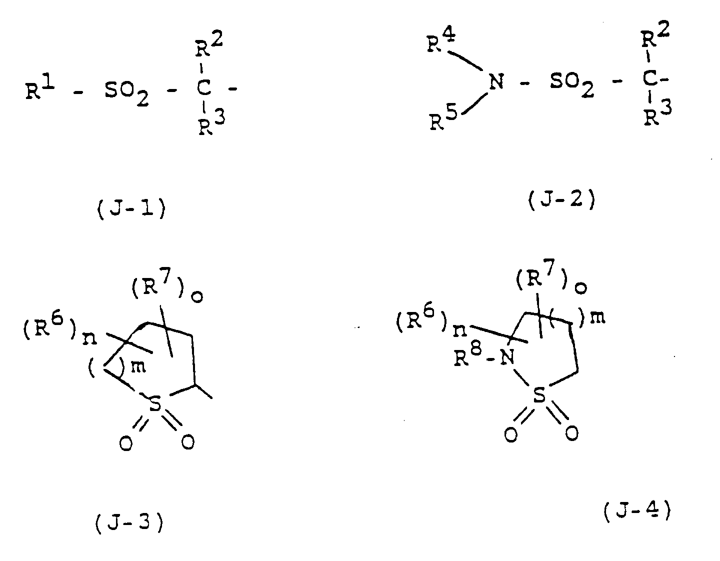 and in these
R¹ is (C₁-C₆)alkyl, (C₂-C₆)alkenyl, (C₂-C₆)alkynyl, the 3 last-mentioned radicals being unsubstituted or monosubstituted or polysubstituted by halogen or monosubstituted or disubstituted by radicals from the group comprising (C₁-C₄)alkoxy, (C₁-C₄)alkylthio and (C₁-C₄)alkoxycarbonyl, or is -(CH₂)ₐ-phenyl, a being 0, 1 or 2 and the phenyl radical being unsubstituted or monosubstituted or polysubstituted by radicals from the group comprising halogen, (C₁-C₄)alkyl, (C₁-C₄)alkoxy, (C₁-C₄)alkylthio, (C₁-C₄)alkylsulfinyl, (C₁-C₄)alkylsulfonyl and (C₁-C₄-alkoxy)carbonyl and radicals which are analogous to the 6 last-mentioned radicals and which are monohalogenated or polyhalogenated in the alkyl moiety,
R² and R³ independently of one another are hydrogen, halogen, (C₁-C₄)alkoxy, (C₁-C₄)alkylthio, (C₁-C₆)alkyl, (C₂-C₆)alkenyl, (C₂-C₆)alkynyl, the 5 last-mentioned radicals being unsubstituted or monosubstituted or polysubstituted by halogen or monosubstituted or disubstituted by radicals from the group comprising (C₁-C₄)alkoxy and (C₁-C₄)alkylthio, or are (C₁-C₄-alkoxy)carbonyl-(C₁-C₃)alkyl, -(CH₂)ₐ-phenyl, a being 0, 1 or 2 and the phenyl radical being unsubstituted or substituted by one or more radicals from the group comprising halogen, (C₁-C₄)alkyl, (C₁-C₄)alkoxy, (C₁-C₄-alkoxy)carbonyl, (C₁-C₄)alkylthio and (C₁-C₄)alkylsulfonyl, the 5 last-mentioned radicals being unsubstituted in the alkyl moiety or monosubstituted or polysubstituted by halogen,
R⁴ and R⁵ independently of one another are hydrogen, (C₁-C₈)alkyl, (C₂-C₈)alkenyl, (C₂-C₈)alkynyl, the 3 last-mentioned radicals being unsubstituted or monosubstituted or polysubstituted by halogen or monosubstituted or disubstituted by (C₁-C₄)alkoxy or (C₁-C₄)alkylthio, or are (C₁-C₄)alkoxycarbonyl-(C₁-C₃)alkyl, -(CH₂)ₐ-phenyl, a being 0, 1 or 2 and the phenyl radical being unsubstituted or monosubstituted or polysubstituted by radicals from the group comprising halogen, (C₁-C₄)alkyl, (C₁-C₄)alkoxy, (C₁-C₄)alkylthio, (C₁-C₄)alkylsulfonyl, the above-mentioned 4 radicals being unsubstituted in the alkyl moiety or monosubstituted or polysubstituted by halogen, and (C₁-C₄-alkoxy)carbonyl, or are (C₁-C₆)alkoxy or di(C₁-C₆)alkylamino, or R⁴ and R⁵ together with the N atom linking them are a heterocyclic ring of the formula 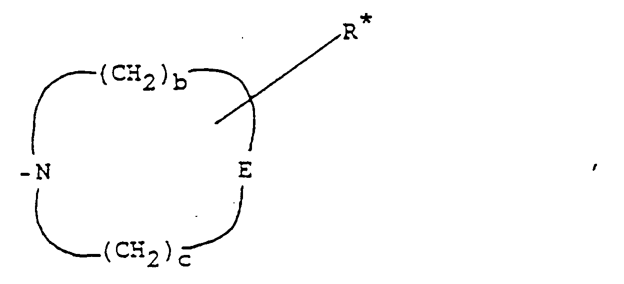 b and c independently of one another being 0, 1, 2 or 3, R* being hydrogen, (C₁-C₄)alkyl, (C₁-C₄-alkoxy)carbonyl or (C₁-C₄-alkoxy)methyl and E being a divalent group of the formula O, S, CH₂ or N-(C₁-C₄)alkyl,
R⁶ and R⁷ independently of one another are halogen, (C₁-C₈)alkyl, (C₂-C₈)alkenyl, (C₂-C₈)alkynyl or (C₁-C₈)alkoxy, the 4 last-mentioned radicals being unsubstituted or monosubstituted or polysubstituted by halogen or monosubstituted or disubstituted by (C₁-C₄)alkoxy or (C₁-C₄)alkylthio, or are (C₁-C₄-alkoxy)-carbonyl-(C₁-C₃)alkyl,
R⁸ is hydrogen, (C₁-C₈)alkyl, (C₂-C₈)alkenyl, (C₂-C₈)alkynyl or (C₁-C₈)alkoxy, the 4 last-mentioned radicals being unsubstituted or monosubstituted or polysubstituted by halogen or monosubstituted or disubstituted by (C₁-C₄)alkoxy or (C₁-C₄)alkylthio, or is (C₁-C₄-alkoxy)carbonyl-(C₁-C₃)alkyl,
R⁹ is hydrogen, (C₁-C₈)alkyl, (C₂-C₈)alkenyl, (C₂-C₈)alkynyl or (C₁-C₈)alkoxy, the 4 last-mentioned radicals being unsubstituted or monosubstituted or polysubstituted by halogen or monosubstituted or disubstituted by (C₁-C₄)alkoxy or (C₁-C₄)alkylthio, or is (C₁-C₄-akoxy)carbonyl-(C₁-C₃)alkyl,
R¹⁰ is hydrogen, (C₁-C₄)alkyl, (C₁-C₄-alkoxy)carbonyl or (C₁-C₄-alkoxy)methyl,
m is 1, 2 or 3,
n and o independently of one another are 0, 1, 2 or 3,
A is a radical of the formulae (A-1) to (A-8) 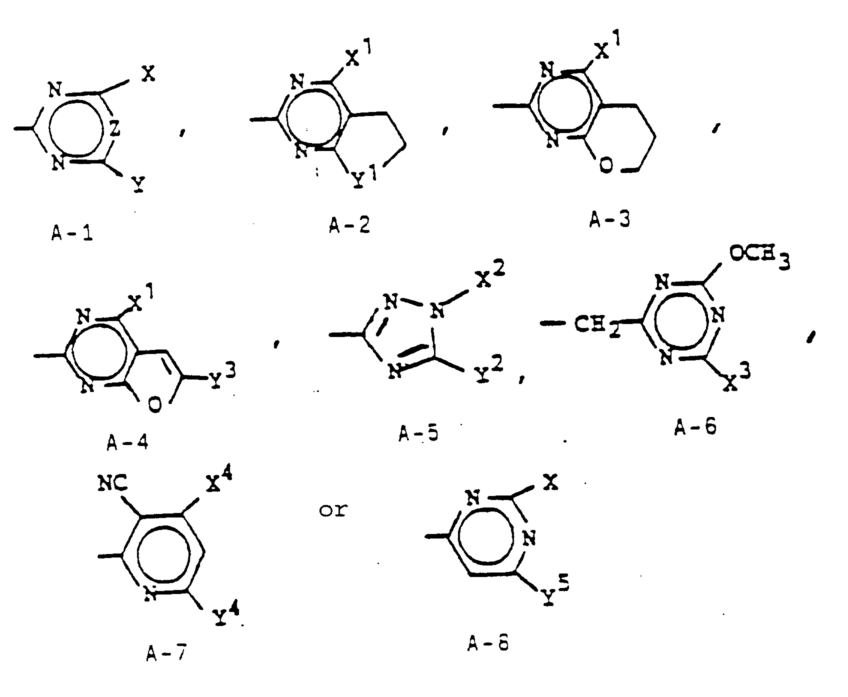
X and Y independently of one another are H, halogen, (C₁-C₃)alkyl, (C₁-C₃)alkoxy or (C₁-C₃)alkylthio, it being possible for the abovementioned alkyl-containing radicals to be monosubstituted or polysubstituted by halogen or monosubstituted or disubstituted by (C₁-C₃)alkoxy or (C₁-C₃)alkylthio, furthermore a radical of the formula NR⁹R¹⁰, (C₃-C₆)cycloalkyl, (C₂-C₄)alkenyl, (C₂-C₄)alkynyl, (C₃-C₄)alkenyloxy or (C₃-C₄)alkynyloxy,
Z is CH or N,
X¹ is CH₃, OCH₃, OC₂H₅ or OCF₂H,
Y¹ is -O- or -CH₂-,
X² is CH₃, C₂H₅ or CH₂CF₃,
Y² is OCH₃, OC₂H₅, SCH₅, SC₂H₅, CH₃ or C₂H₅,
X³ is CH₃ or OCH₃,
Y³ is H or CH₃,
X⁴ is CH₃, OCH₃, OC₂H₅, CH₂OCH₃ or Cl,
Y⁴ is CH₃, OCH₃, OC₂H₅ or Cl,
Y⁵ is CH₃, C₂H₅, OCH₃ or Cl,
W is O or S and
R is hydrogen or CH₃.

2. A compound and salts thereof, as claimed in claim 1, wherein A is a radical of the formula (A-1).

3. A compound and salts thereof, as claimed in claim 1 or 2, wherein
R¹ is (C₁-C₄)alkyl, R² and R³ independently of one another are hydrogen, (C₁-C₄)alkyl or (C₂-C₄)alkenyl, R⁴ and R⁵ independently of one another are hydrogen, (C₁-C₄)alkyl, (C₁-C₄)alkoxy, (C₂-C₄)alkenyl, or R⁴ and R⁵ together are a heterocyclic radical of the formula 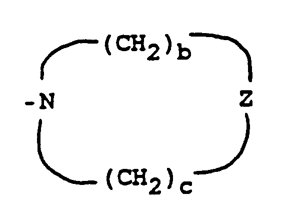 b and c independently of one another being 0, 1, 2 or 3, the total of b+c being the number 3 or 4, and Z being O or CH₂, R⁶ and R⁷ independently of one another are (C₁-C₃)alkyl, R⁸ is (C₁-C₄)alkyl or (C₂-C₄)alkenyl, R⁹ and R¹⁰ independently of one another are hydrogen or (C₁-C₄)alkyl, m is 1 or 2, n and o independently of one another are 0, 1 or 2, preferably 0 or 1, A is a radical of the formula (A-1), X and Y independently of one another are halogen, (C₁-C₄)alkyl or (C₁-C₄)alkoxy, it being possible for the two last-mentioned radicals to be monosubstituted or polysubstituted by halogen, and W is an oxygen atom.

4. A process for the preparation of compounds of the formula (I) or salts thereof, as claimed in one or more of claims 1 to 3, which comprises reacting
a) a compound of the formula (II)
J - SO₂NH₂ (II)
in which J is as defined in formula (I),
with a heterocyclic (thio)carbamate of the formula (III) 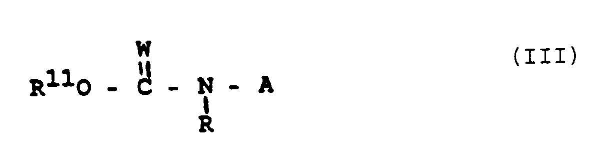 in which R¹¹ is (C₁-C₆)alkyl, (C₁-C₄)haloalkyl or phenyl which is unsubstituted or monosubstituted or polysubstituted by radicals from the group comprising halogen, (C₁-C₄)alkyl and NO₂,
and A, R and W have the meanings as in formula (I),
b) a sulfonyl carbamate of the formula (IV) 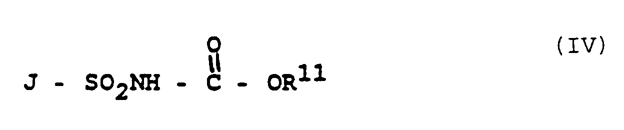 in which R¹¹ is as defined under a),
with an amino heterocycle of the formula (V) 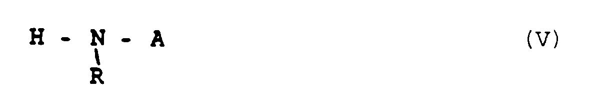 in which A and R are as defined in formula (I), or
c) a sulfonyl isocyanate of the formula (VI)
J - SO₂NCO (VI)
in which J is as defined in formula (I),
with an amino heterocycle of the formula (V).

5. A herbicidal or plant-growth-regulating agent, which contains a compound of the formula (I) or a salt thereof, as claimed in one or more of claims 1 to 3, besides customary formulation auxiliaries.

6. The use of compounds of the formula (I) or salts thereof, as claimed in one or more of claims 1 to 3, as herbicides or plant growth regulators.

7. A method of selectively controlling harmful plants, or of regulating the growth of plants, which comprises applying an effective amount of a compound or salts thereof, as defined in one or more of claims 1 to 3, to the plants, seeds of the plants, or the area on which they grow.

## Revendications

1. Composés de formule générale (I) ou leurs sels 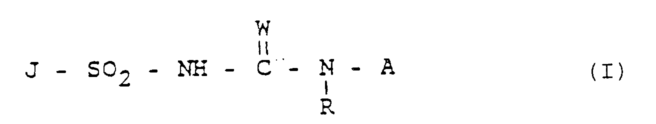 dans laquelle J représente un reste de formule (J-1)-(J-4) 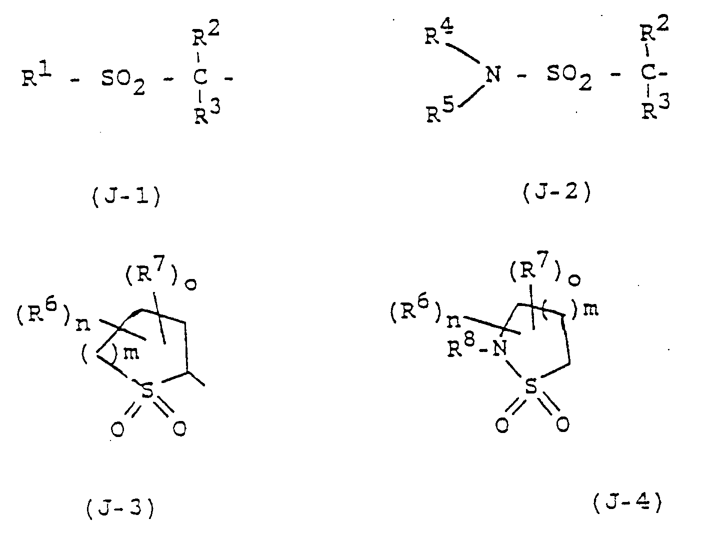 et dans lesquelles
R¹ représente des groupes alkyle en C₁-C₆, alcényle en C₂-C₆, alcynyle en C₂-C₆, les trois derniers restes pouvant être non substitués ou substitués une ou plusieurs fois par des halogènes ou une ou plusieurs fois par des restes pris parmi les groupes alcoxy en C₁-C₄, (alkyl en C₁-C₄)thio et (alcoxy en C₁-C₄)carbonyle, ou représente -(CH₂)ₐ-phényle, a valant 0, 1 ou 2 et le reste phényle étant non substitué ou une ou plusieurs fois substitué par des restes pris parmi les groupes halogènes, alkyle en C₁-C₄, alcoxy en C₁-C₄, (alkyl en C₁-C₄)thio, (alkyl en C₁-C₄)sulfinyle, (alkyl en C₁-C₄)sulfonyle et (alcoxy en C₁-C₄)carbonyle et des restes de façon analogue aux 6 derniers restes cités qui sont halogénés une ou plusieurs fois dans la partie alkyle,
R², R³ représentent, indépendamment l'un de l'autre, un atome d'hydrogène, un atome d'halogène, alcoxy en C₁-C₄, (alkyl en C₁-C₄)thio, alkyle en C₁-C₆, alcényle en C₂-C₆, alcynyle en C₂-C₆, les 5 derniers reste cités pouvant être non substitués ou substitués une ou plusieurs fois par des atomes d'halogènes ou une ou deux fois par des restes pris parmi les groupes alcoxy en C₁-C₄ et (alkyl en C₁-C₄)thio, représentent aussi (alcoxy en C₁-C₄)-carbonylalkyle en C₁-C₃, -(CH₂)ₐ-phényle, a valant 0, 1 ou 2 et le reste phényle étant non substitué ou substitué par un ou plusieurs restes pris parmi les atomes d'halogènes, les groupes alkyle en C₁-C₄, alcoxy en C₁-C₄, (alcoxy en C₁-C₄)carbonyle, (alkyl en C₁-C₄)thio et (alkyl en C₁-C₄)sulfonyle, les derniers 5 restes cités étant non substitués ou substitués une ou plusieurs fois par des atomes d'halogène dans la partie alkyle,
R⁴, R⁵, représentent, indépendamment l'un de l'autre, un atome d'hydrogène, alkyle en C₁-C₈, alcényle en C₂-C₈, alcynyle en C₂-C₈, les 3 derniers restes cités étant non substitués ou substitués une ou plusieurs fois par des atomes d'halogène ou une ou plusieurs fois par des groupes alcoxy en C₁-C₄ ou (alkyl en C₁-C₄)thio, représentent aussi (alcoxy en C₁-C₄)carbonylalkyle en C₁-C₃, -(CH₂)ₐ-phényle, a valant 0, 1 ou 2 et le reste phényle étant non substitué ou substitué une ou plusieurs fois par des atomes d'halogène, des groupes alkyle en C₁-C₄, alcoxy en C₁-C₄, (alkyl en C₁-C₄)thio, (alkyl en C₁-C₄)sulfonyle, les 4 derniers restes cités étant non substitués ou substitués dans la partie alkyle une ou plusieurs fois par des atomes d'halogènes, et est substitué dans la partie alkyle par (alcoxy en C₁-C₄)-carbonyle, alcoxy en C₁-C₆ ou di(alkyl en C₁-C₆)-amino ou R⁴ et R⁵, ensemble avec l'atome de N qui les relie, forment un hétérocycle de formule 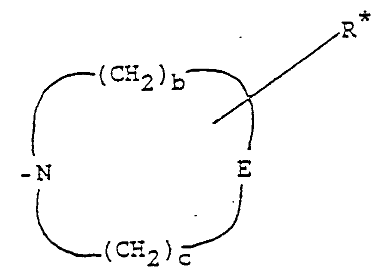 b et c, indépendamment l'un de l'autre, valant 1, 1, 2 ou 3, R* représentant un atome d'hydrogène, des groupes alkyle en C₁-C₄, (alcoxy en C₁-C₄)carbonyle ou (alcoxy en C₁-C₄)méthyle et E un groupe bivalent de formule O, S, CH₂ ou N-(alkyle en C₁-C₄),
R⁶, R⁷, ondépendamment l'un de l'autre, représente un atome d'halogène des groupes alkyle en C₁-C₈, alcényle en C₂-C₈, alcynyle en C₂-C₈ ou alcoxy en C₁-C₈,, les 4 derniers restes cités étant non substitués ou substitués une ou plusieurs fois par des atomes d'halogènes ou une ou deux fois par des groupes alcoxy en C₁-C₄ ou (alkyl en C₁-C₄)thio, ou (alcoxy en C₁-C₄)-carbonyl-(alkyle en C₁-C₃),
R⁸ représente un atome d'hydrogène, des groupes alkyle en C₁-C₈, alcényle en C₂-C₈, alcynyle en C₂-C₈ ou alcoxy en C₁-C₈, les 4 derniers restes cités sont non substitués ou substitués une ou deux fois par des atomes d'halogènes ou une ou deux fois par des groupes alcoxy en C₁-C₄ ou (alkyl en C₁-C₄)thio, ou (alcoxy en C₁-C₄)carbonyl-(alkyle en C₁-C₃),
R⁹ représente un atome d'hydrogène, des groupes alkyle en C₁-C₈, alcényle en C₂-C₈, alcynyle en C₂-C₈ ou alcoxy en C₁-C₈, les 4 derniers restes cités étant non substitués ou substitués une ou plusieurs fois par des atomes d'halogènes ou une ou deux fois par des groupes alcoxy en C₁-C₄ ou (alkyl en C₁-C₄)thio, ou (alcoxy en C₁-C₄)carbonyl-(alkyle en C₁-C₃),
R¹⁰ représente un atome d'hydrogène, des groupes alkyle en C₁-C₄, (alcoxy en C₁-C₄)carbonyle ou (alcoxy en C₁-C₄)méthyle,
m vaut 1, 2 ou 3,
n, o indépendamment l'un de l'autre, valent 0, 1, 2 ou 3,
A représente un reste de formule (A-1) à (A-8) 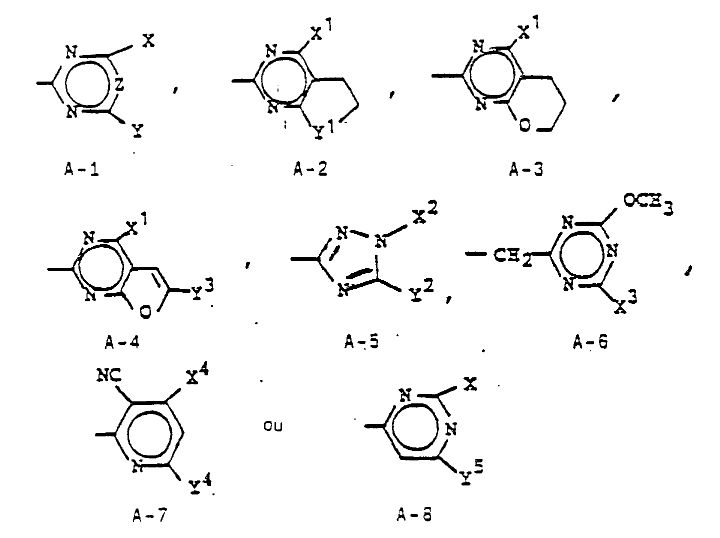
X, Y représentent un atome d'hydrogène, un atome d'halogène, des groupes alkyle en C₁-C₃, alcoxy en C₁-C₃ ou (alkyl en C₁-C₃)thio, les restes alkylés précités pouvant être substitués une ou plusieurs fois par des atomes d'halogènes ou une ou deux fois par des groupes alcoxy en C₁-C₃ ou (alkyl en C₁-C₃)thio, en plus représentent un reste NR⁹R¹⁰, des groupes cycloalkyle en C₃-C₆, alcényle en C₂-C₄, alcynyle en C₂-C₄, alcényloxy en C₃-C₄ ou alcynyloxy en C₃-C₄, de préférence à la condition, que les deux restes ne soient pas simultanément des restes pris parmi les atomes d'halogènes, les groupes alkylthio, les restes NR⁹R¹⁰, les groupes alcényule et alcynyle,
Z représente CH ou N, de préférence CH,
X¹ représente CH₃, OCH₃, OC₂H₅ ou OCF₂H,
Y¹ représente -O- ou -CH₂-,
X² représente CH₃, C₂H₅ ou CH₂CF₃,
Y² représente OCH₃, OC₂H₅, SCH₅, SC₂H₅, CH₃ ou C₂H₅,
X³ représente CH₃ ou OCH₃,
Y³ représente H ou CH₃,
X⁴ représente CH₃, OCH₃, OC₂H₅, CH₂OCH₃ ou Cl,
Y⁴ représente CH₃, OCH₃, OC₂H₅ ou Cl,
Y⁵ représente CH₃, C₂H₅, OCH₃ ou Cl,
W représente O ou S et
R représente un atome d'hydrogène ou CH₃.

2. Composés et leurs sels selon la revendication 1, caractérisés en ce que A représente un reste de formule (A-1).

3. Composés et leurs sels selon la revendication 1 ou 2, caractérisés en ce que
R¹ = alkyle en C₁-C₄, R² et R³, indépendamment l'un de l'autre, représentent un atome d'hydrogène, des groupes alkyle en C₁-C₄ ou alcényle en C₁-C₄, R⁴ et R⁵, indépendamment l'un de l'autre représentent un reste hétérocyclique de formule 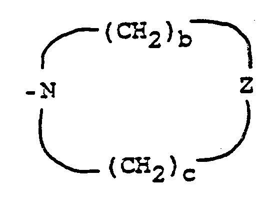 b et c, indépendamment l'un de l'autre, représentent 0, 1, 2 ou 3, la somme de b+c étant 3 ou 4 et Z représente O ou CH₂, R⁶ et R⁷, indépendamment l'un de l'autre, représentent alkyle en C₁-C₃, R⁸ représente alkyle en C₁-C₄ ou alcényle en C₁-C₄, R⁹ et R¹⁰, indépendamment l'un de l'autre, représentent un atome d'hydrogène ou un groupe alkyle en C₁-C₄, m vaut 1 ou 2, n et o, indépendamment l'un de l'autre, valent 0, 1 ou 2, de préférence 0 ou 1, A représente un reste de formule (A-1), X et Y, independamment l'un de l'autre, représentent un atome d'halogène, des groupes alkyle en C₁-C₄ ou alcoxy en C₁-C₄, les deux derniers restes cités pouvant être substitués une ou plusieurs fois par des atomes d'halogènes, et W représente un atome d'oxygène.

4. Procédé de préparation de composés de formule (I) ou leurs sels selon une ou plusieurs des revendications 1 à 3, caractérisé en ce que l'on fait réagir
a) un composé de formule (II)
J-SO₂NH₂ (II)
dans laquelle J est défini comme dans la formule (I),
avec un (thio)-carbamate hétérocyclique de formule (III) 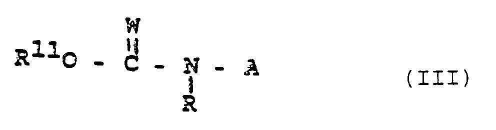 dans laquelle R¹¹ = alkyle en C₁-C₆, halogéno-alkyle en C₁-C₄ ou phényle, qui est non substitué ou substitué une ou plusieurs fois par des restes pris dans le groupe comportant des atomes d'halogènes, des groupes alkyle en C₁-C₄ et NO₂,
et A, R et W ont les significations comme dans la formule (I)
b) un sulfonylcarbamate de formule (IV) 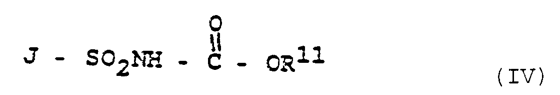 dans laquelle R¹¹ est défini comme au point a),
avec un hétérocycle aminé de formule (V), 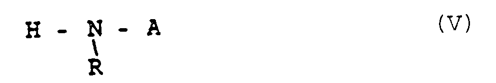 dans laquelle A et R sont définis comme dans la formule (I),
ou
c) un sulfonylisocyanate de formule (VI)
J-SO₂NCO (VI)
dans laquelle J est défini comme dans la formule (I),
avec un hétérocycle aminé de formule (V).

5. Agents herbicides ou régulateurs de croissance végétale caractérisés en ce qu'ils contiennent un composé de formule (I) ou leur sel selon une ou plusieurs des revendications 1 à 3, outre des adjuvants de formulation courants.

6. Utilisation des composés de formule (I) ou leurs sels selon une ou plusieurs des revendications 1 à 3 comme herbicides ou régulateurs de croissance végétale.

7. Procédé de lutte sélective contre des plantes nuisibles ou pour la régulation de la croissance de plantes, caractérisé en ce que l'on applique une quantité efficace d'un composé défini selon une ou plusieurs des revendications 1 à 3 ou de ses sels sur les plantes, la semence ou la surface cultivable.
